(19) Europäisches Patentamt
European Patent Office
Office européen des brevets

(11) **EP 3 152 556 B1**

(12) **EUROPEAN PATENT SPECIFICATION**

(45) Date of publication and mention
of the grant of the patent:
**11.03.2020 Bulletin 2020/11**

(51) Int Cl.:
**G01N 27/02** (2006.01)    **A61B 5/053** (2006.01)
**A61B 5/11** (2006.01)    **A61B 5/00** (2006.01)
**A61B 5/021** (2006.01)    **A61B 5/024** (2006.01)
**A61B 5/08** (2006.01)

(21) Application number: **15724678.6**

(22) Date of filing: **27.05.2015**

(86) International application number:
**PCT/EP2015/061640**

(87) International publication number:
**WO 2015/185398 (10.12.2015 Gazette 2015/49)**

(54) **APPARATUS AND METHODS THAT USE MAGNETIC INDUCTION SPECTROSCOPY TO MONITOR TISSUE FLUID CONTENT**

VORRICHTUNG UND VERFAHREN UNTER VERWENDUNG VON MAGNETISCHER INDUKTIONSSPEKTROSKOPIE ZUR ÜBERWACHUNG DES GEWEBEFLÜSSIGKEITSGEHALTS

APPAREIL ET PROCÉDÉS UTILISANT LA SPECTROSCOPIE À INDUCTION MAGNÉTIQUE POUR SURVEILLER UN CONTENU DE FLUIDE TISSULAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.06.2014 EP 14170979**

(43) Date of publication of application:
**12.04.2017 Bulletin 2017/15**

(73) Proprietor: **Koninklijke Philips N.V.**
**5656 AE Eindhoven (NL)**

(72) Inventors:
• **BEZEMER, Rick**
**5656 AE Eindhoven (NL)**
• **BONGERS, Edwin Gerardus Johannus Maria**
**5656 AE Eindhoven (NL)**

(74) Representative: **van Iersel, Hannie**
**Philips Intellectual Property & Standards**
**High Tech Campus 5**
**5656 AE Eindhoven (NL)**

(56) References cited:
**WO-A1-2010/113067**    **GB-A- 2 500 216**
**US-A1- 2010 127 705**

• **ZULKARNAY ZAKARIA ET AL: "Advancements in Transmitters and Sensors for Biological Tissue Imaging in Magnetic Induction Tomography", SENSORS, vol. 12, no. 12, 29 May 2012 (2012-05-29), pages 7126-7156, XP055149580, ISSN: 1424-8220, DOI: 10.3390/s120607126**
• **ZHENG XU ET AL: "A multi-channel magnetic induction tomography measurement system for human brain model imaging", PHYSIOLOGICAL MEASUREMENT, vol. 30, no. 6, 2 June 2009 (2009-06-02), pages S175-S186, XP055201822, ISSN: 0967-3334, DOI: 10.1088/0967-3334/30/6/S12**
• **H SCHARFETTER ET AL: "Tracking of object movements for artefact suppression in Magnetic Induction Tomography (MIT)", JOURNAL OF PHYSICS: CONFERENCE SERIES, vol. 224, 1 April 2010 (2010-04-01), page 012040, XP055201780, DOI: 10.1088/1742-6596/224/1/012040**

**Description**

TECHNICAL FIELD OF THE INVENTION

The invention relates to apparatus and methods that use magnetic induction spectroscopy (MIS) to monitor the fluid content of tissue in a human or animal body, and in particular to apparatus and methods that use MIS that are robust against movement of the apparatus and/or tissue, and/or robust against the low signal levels obtained from the tissue.

BACKGROUND TO THE INVENTION

The total amount of water in a man of average weight is approximately 40 litres, which is around 60% of his total body weight. Around two-thirds of the total amount of water in a man is intracellular fluid (i.e. within the cells of the body) and one-third is extracellular fluid i.e. outside the cells of the body). The extracellular fluid is, in turn, divided over three 'compartments' or 'spaces'. The first compartment is the intravascular space, which comprises blood cells and plasma (making up around 4% of total body weight). The second compartment is the interstitial space or tissue space, which is filled with fluid that surrounds the cells (making up around 16% of total body weight). The third space is space in the body where fluid does not normally collect, or where any significant fluid collection is physiologically non-functional or even harmful due to depressed oxygen diffusion. Significant examples of the third type of space include the peritoneal cavity and pleural cavity.

Under normal conditions, the amount of fluid in the body is well maintained in terms of both volume and electrolyte concentrations through processes like drinking and urine production. However, fluid balance may be disturbed due to a variety of reasons. Fluid shifts between tissue and blood occur through changes in hydrostatic pressure gradients and/or osmotic pressure gradients. Water moves passively from one compartment into another until the hydrostatic and osmotic pressure gradients balance each other. However, many medical conditions can cause fluid shifts that alter the fluid volumes and electrolyte concentrations in the different compartments. For example, if fluid moves out of the blood vessels, hypovolemia might arise and result in hypotension and shock. If fluid accumulates in tissues, oxygen diffusion is hampered which leads to organ hypoxia and consequent dysfunction. Fluid shifts may be compensated by fluid replacement or diuretics.

Fluid-related complications can roughly be divided into four categories: (1) whole-body dehydration due to insufficient fluid intake; (2) whole body-dehydration due to onset and progression of hypovolemia caused by bleeding or other excessive fluid losses; (3) whole-body over-hydration due to excessive fluid therapy to treat hypovolemia; and (4) local or whole-body edema formation due to a specific underlying pathology including heart, kidney, or liver dysfunction, sepsis, diabetes, hypertension, and pregnancy. Complications associated with under- and over-hydration start when the fluid balance is deviating from 'normal' levels by, for example, ±2%. Dehydration of >15% is often lethal and over-hydration of >15% is associated with severe organ edema and consequent organ dysfunction, co-morbidities, and potential mortality.

Thus there is a clinical need for a reliable, easy-to-use, preferably continuous, and non-invasive apparatus and method for monitoring the fluid content of tissue (e.g. a specific part of the body such as an arm or leg, or the whole body) in order to provide an early warning for changes in a patient's hydration status. Furthermore, it is desirable to provide non-invasive apparatus for measuring other physiological characteristics of a patient, such as heart rate.

Currently, the monitoring of the local fluid distribution or fluid content in human tissues non-invasively presents a difficult task. Established methods are either complicated and/or costly (e.g. tracer dilution, imaging methods) or exhibit poor reproducibility (bioimpedance spectroscopy, BIS).

US 2010/0127705 describes a method and apparatus for magnetic induction tomography. The papers entitled "Advancements in Transmitters and Sensors for Biological Tissue Imaging in Magnetic Induction Tomography" by Zulkarnay Zakaria et al. in Sensors, vol. 12, no. 12, 29 May 2012, pages 7126-7156 and "A multi-channel magnetic induction tomography measurement system for human brain model imaging" by Zheng Xu et al, in PHYSIOLOGICAL MEASUREMENT, vol. 30, no. 6, 2 June 2009 (2009-06-02), pages S175-S186 also describe magnetic induction tomography.

Magnetic induction spectroscopy (MIS) is a relatively new method for measuring the electrical conductivity of biological tissue via the perturbation of an alternating magnetic field at multiple frequencies. MIS is described, for example, in the paper "Over-Hydration Detection in Brain by Magnetic Induction Spectroscopy" by Gonzalez et al., International Conference on Electrical Bioimpedance, Journal of Physics: Conference Series 224 (2010). This paper describes the non-invasive detection of tissue edema using multifrequency induced magnetic fields, and in particular indicates that measuring induction phase shift throughout the bulk of the tissue in time and in a broad range of frequencies could be used as an alternative technique for detection of tissue edema formation.

One of the main advantages of MIS over BIS is that MIS examines the tissue in a fixed volume, i.e. within the magnetic field, while the measurement volume of BIS is not defined since the distribution of the current depends on the path of least resistance. This has two important implications. Firstly, MIS, in principle, could be a fully contactless method for assessing tissue fluid content, and secondly MIS can allow control over the measurement depth, since the penetration depth of the excitation field can be controlled.

Currently, MIS has only been used to detect brain and lung edema, and it has not been used for the assessment of the general hydration status (e.g. under- or over-hydration) of patients. A MIS-based technology for providing non-invasive fluid management support by monitoring tissue composition (e.g. tissue water content) and/or monitoring vital signs (e.g. heart rate) would be useful.

However, using MIS to monitor tissue fluid content comes with some practical challenges related to the low signal levels from the tissue and relatively large noise levels due to movement artefacts (e.g. movement of the tissue under examination and/or movement of the components of the MIS apparatus).

Therefore there is a need for apparatus and methods for using MIS to monitor the fluid content of tissue in a human or animal body that are robust against movement of the apparatus and/or tissue, and/or robust against the low signal levels obtained from the tissue.

## SUMMARY OF THE INVENTION

A first aspect provides an apparatus for using magnetic induction spectroscopy to determine a measure of the fluid content of a tissue sample of a subject according to claim 1.

A second aspect provides a method of using magnetic induction spectroscopy to determine a measure of the fluid content of a tissue sample of a subject according to claim 8.

## BRIEF DESCRIPTION OF THE DRAWINGS

Exemplary embodiments of the invention will now be described, by way of example only, with reference to the following drawings, in which:

Figure 1 illustrates the principle of using magnetic induction spectroscopy to obtain tissue fluid measurements;
Figures 2(a), (b) and (c) illustrate known magnetic induction spectroscopy, MIS, apparatus;
Figure 3 is a block diagram of a first embodiment of a MIS apparatus according to the invention;
Figure 4 is an illustration of the interconnection of coils in the MIS apparatus of Figure 3;
Figure 5 is a flow chart illustrating an exemplary method of using MIS to monitor tissue fluid content according to the invention;
Figure 6 is a block diagram of a second embodiment of a MIS apparatus according to the invention;
Figure 7 is a block diagram of a first example of a MIS apparatus according to a first alternative implementation, not falling within the scope of the claims;
Figure 8 is a block diagram of a second example of a MIS apparatus according to the first alternative implementation, not falling within the scope of the claims;
Figure 9 is a block diagram of a first example of a MIS apparatus according to a second alternative implementation, not falling within the scope of the claims;
Figure 10 is a flow chart illustrating a method of using MIS to monitor tissue fluid content according to the second alternative implementation, not falling within the scope of the claims;
Figure 11 is a block diagram of a second example of a MIS apparatus according to the second alternative implementation, not falling within the scope of the claims;
Figure 12 is a block diagram of a third example of a MIS apparatus according to the second alternative implementation, not falling within the scope of the claims;
Figure 13 is a block diagram of a first example of a MIS apparatus according to a third alternative implementation, not falling within the scope of the claims;
Figure 14 is a flow chart illustrating a method of using MIS to monitor tissue fluid content according to the third alternative implementation;
Figure 15 is a block diagram of a second example of a MIS apparatus according to the third alternative implementation, not falling within the scope of the claims;
Figure 16 is a block diagram of a MIS apparatus according to the second and third alternative implementation, not falling within the scope of the claims;
Figure 17 is a block diagram of another MIS apparatus according to the second and third alternative implementation, not falling within the scope of the claims; and
Figure 18 is a flow chart illustrating a general method according to the invention.

DETAILED DESCRIPTION OF THE PREFERRED EMBODIMENTS

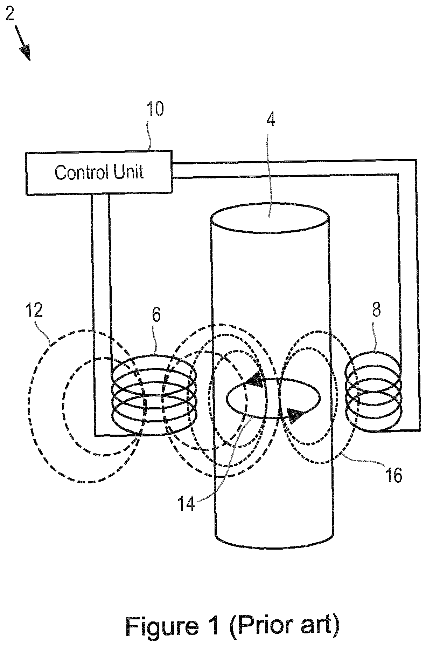

Figure 1 illustrates the principle of using magnetic induction spectroscopy to obtain tissue fluid measurements. In particular, Figure 1 illustrates a conventional magnetic induction spectroscopy (MIS) apparatus 2 that is being used to measure the fluid content of a tissue sample 4. The tissue sample 4 can be any part of a subject, including part of a limb, such as an arm, leg, finger, toe, wrist or ankle, or a larger part of the body of a patient or animal, such as the torso or chest.

The MIS apparatus 2 comprises an excitation coil 6 and a receiver coil 8 that are connected to a control unit 10. The excitation coil 6 is arranged adjacent to the tissue sample 4 of the subject and the receiver coil 8 is arranged generally on the opposite side of the tissue sample 4 to the excitation coil 6. The excitation coil 6 and receiver coil 8 are arranged so that they are in the same orientation with respect to each other - i.e. they are arranged so that their axes are parallel to each other.

The control unit 10 drives the excitation coil 6 with an excitation signal in the form of an alternating current (typically at a frequency in the range of 1-10 MHz) in order to generate a magnetic field. The alternating magnetic field generated by the excitation coil 6 is shown by magnetic field lines 12 in Figure 1. The alternating magnetic field 12 generates an eddy current 14 in the tissue sample 4. The strength of the eddy current 14 depends on the strength of the excitation field 12 and the conductivity of the sample 4. For biological tissue, the conductivity of the sample 4 depends on the amount of water (including the amount of blood) in the tissue sample 4 within the excitation field 12. The eddy current 14 is sensed by the receiver coil 8, which is used to detect the magnetic field created by the generated eddy current 14. Lines 16 illustrate the magnetic field induced by the eddy current 14. In particular the magnetic field 16 generated by the eddy current 14 generates a current in the receiver coil 8, and this current is processed by the control unit 10 to determine the, or an indication of the, fluid content of the tissue sample 4.

It will be appreciated that low tissue water content results in low tissue conductivity and magnetic inductance and this, in turn, results in a weaker magnetic field 16 generated by the eddy current 14, which is sensed by the receiver coil 8. Similarly, higher tissue water content results in higher tissue conductivity and magnetic inductance and thus a stronger eddy current 14 and stronger magnetic field 16. It will be appreciated that pulsatile beat-to-beat changes in tissue blood volume result in pulsatile beat-to-beat changes in tissue conductivity.

With the excitation coil 6 and receiver coil 8 arranged in the same orientation, there will be coil crosstalk between the excitation coil 6 and receiver coil 8 which will influence the measurement and is affected by the mechanical stability and thermal variations of the apparatus 2. That is, the excitation magnetic field 12 may directly induce a current in the receiver coil which can obscure the signal corresponding to the current induced by the eddy current 14 in the tissue sample 4.

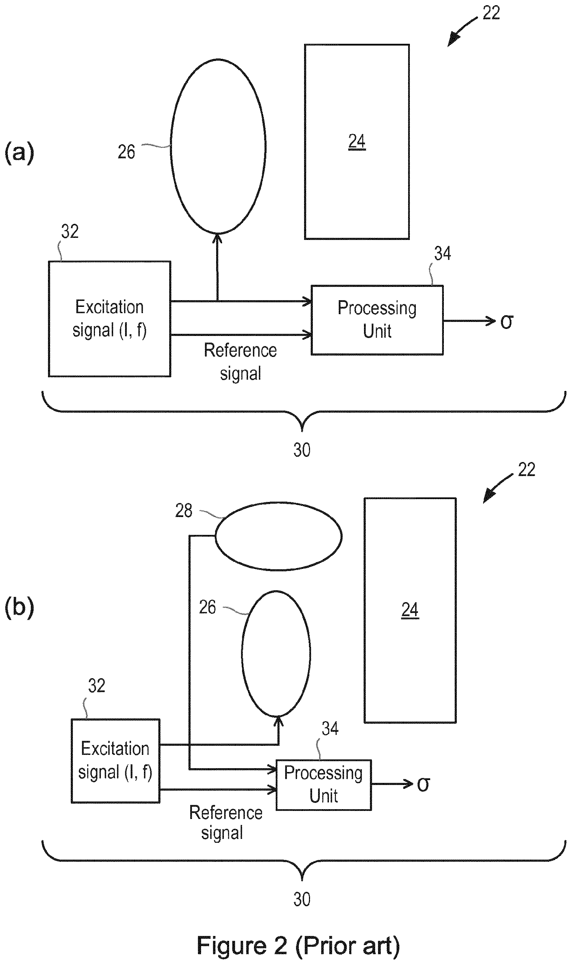

In some known implementations, the problem of coil crosstalk can be avoided by omitting the receiver coil 8 and sensing the magnetic field 16 generated by the eddy current 14 in the tissue sample 4 using the excitation coil 6. Figure 2(a) is a simplified illustration of an apparatus 22 according to this implementation. The apparatus 22 in Figure 2(a) is similar to apparatus 2 shown in Figure 1, and is for measuring the fluid content of a tissue sample 24 and comprises an excitation coil 26 arranged near to the sample 24 and a control unit 30 that is connected to the excitation coil 26. The control unit 30 comprises excitation coil driving circuitry 32 that generates an alternating current having magnitude I and frequency f for driving the excitation coil 26 to generate a magnetic field, and a processing unit 34 that receives the voltage signal induced in the excitation coil 26 by the eddy current in the tissue sample 24 and determines an indication of the tissue fluid content (e.g. a measure of the conductivity of the tissue sample 24) from the voltage signal.

In some other known implementations, as illustrated in Figure 2(b) (which uses the same reference numerals as Figure 2(a)), the problem of coil crosstalk is overcome by positioning the receiver coil 28 such that it is minimally sensitive to the large excitation field from the excitation coil 26 but still sensitive to the small magnetic field from the eddy current in the biological tissue sample 24. In particular, the receiver coil 28 can be arranged orthogonally to the excitation coil 26 (e.g. the receiver coil 28 can be arranged so that it's axis is at 90° with respect to the axis of the excitation coil 26). This configuration is called a Zero Flow Coil (ZFC). In this case, the signal (voltages) obtained in the receiver coil 28 will originate in part from coil crosstalk (which is substantially reduced compared to when the axes of the coils 26, 28 are parallel) and from the current induced in the coil 28 by the indirect magnetic field from the tissue sample 24.

With both of the above implementations it has been found that a good way to detect changes in tissue conductivity (and thus tissue fluid content) using MIS is to perform a phase measurement between the excitation coil 6, 26 and the receiver coil 8, 28. The phase angle $\varphi$ corresponds to the conductivity of the tissue and is the parameter of interest. During relative movements between the tissue sample 24 and the coils this phase angle $\varphi$ stays fairly constant and remains mainly dependent on the tissue conductivity. To enable this measurement, the processing unit 34 receives a reference signal from the excitation coil driving circuitry 32 that corresponds to the voltage signal used to drive the excitation coil 26 and determines the phase angle $\varphi$ from the reference signal and the voltage signal from the receiver coil 28.

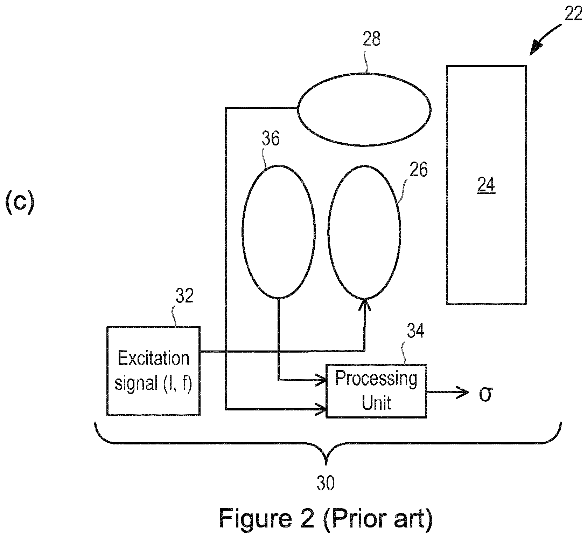

It has been appreciated that it is important to have a phase measurement with very low coil crosstalk and excellent overall phase stability over time. A known approach to eliminate phase influence of the excitation coil driving circuitry 32 is to measure the phase difference $\Delta_\varphi$ between the receiver coil 28 and a reference coil that has a strong mutual induction with the excitation coil 26. Figure 2(c) illustrates an apparatus 22 according to this approach. In this approach, the receiver coil 28 is arranged orthogonally to the excitation coil 26 as in Figure 2(b) and a reference coil 36 is provided that is connected to the processing unit 34. The reference coil 36 is arranged near to the excitation coil 28 and in the same orientation as the excitation coil 28 to maximise the coil crosstalk. The processing unit 34 can measure the coil crosstalk using the signal from the reference coil 36 and subtract this from the voltage signal from the receiver coil 28 to improve the measure of the fluid content of the sample 24.

With the arrangement in Figure 2(c), the effects of variations in the excitation coil driving circuitry 32 and excitation coil 34, e.g. due to temperature variations, are strongly suppressed. By matching the reference coil 36 and receiver coil 28 some of the drift in the detection system can be compensated. In addition, with a strategically placed reference coil 36, relative motion between the tissue sample 24 and the coils only results in a common gain change while the phase will stay constant. Thus, it is important to make sure that the reference coil 36 has as little mutual induction to the tissue sample 24 as possible. Effectively, this is similar to a magnetic gradiometer.

A remaining issue with the arrangement in Figure 2(c) is that the reference coil 36 is still sensitive to the magnetic field generated by the eddy current in the tissue sample 24.

To address this issue, according to the invention a second excitation coil is provided in the MIS apparatus that is paired with the reference coil, with the coupling between the second excitation coil and the reference coil matching the coupling between the (first/main) excitation coil and the receiver coil. In this configuration, the coupling between the excitation coil and receiver coil is far from zero, but this coupling is matched with the coupling between the second excitation coil and the reference coil.

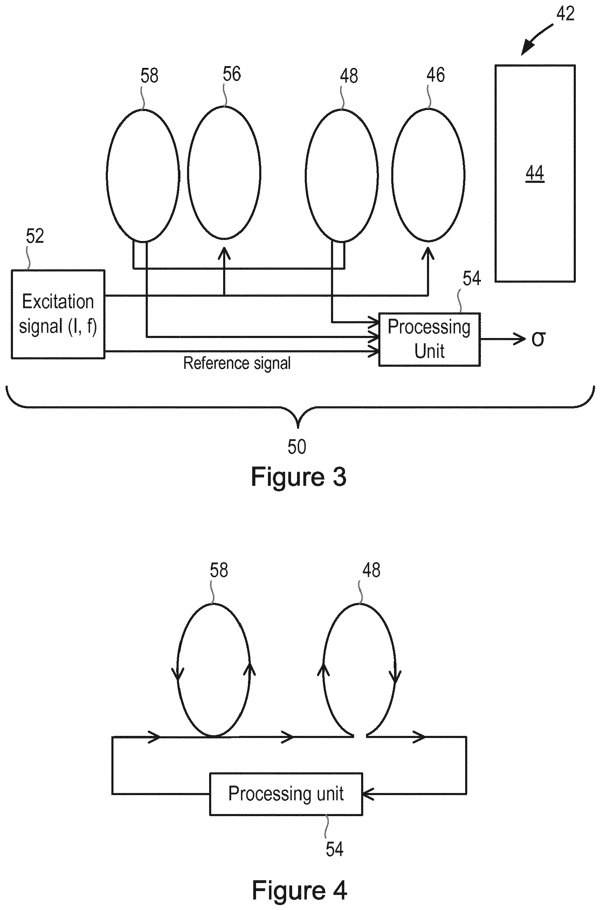

A first embodiment of a MIS apparatus 42 according to the invention is shown in Figure 3. The MIS apparatus 42 is for use in measuring the fluid content of a tissue sample 44, such as an arm, leg, torso, chest, etc. of a patient or animal.

The MIS apparatus 42 comprises an excitation coil 46 and a receiver coil 48 that are connected to a control unit 50. The excitation coil 46 is arranged adjacent to the tissue sample 44 (e.g. no more than a few centimetres (e.g. 5 cm) from the surface of the tissue sample 44) and the receiver coil 48 is also arranged adjacent to the tissue sample 44 (the receiver coil 48 should also be no more than a few centimetres (e.g. 5 cm) from the surface of the tissue sample 44). In some implementations the receiver coil 48 can be arranged generally on the opposite side of the tissue sample 44 to the excitation coil 46, although it will be appreciated that arranging the receiver coil 48 in this way is not required. All that is required is that the receiver coil 48 is placed somewhere close to the tissue sample 44 so that it can measure the eddy current induced in the tissue sample 44 by the excitation coil 46. In this embodiment the excitation coil 46 and receiver coil 48 are arranged so that they are in the same orientation with respect to each other - i.e. they are arranged so that their axes are parallel to each other.

The diameters of the excitation coil 46 and receiver coil 48 and the number of windings depend on the size/area of the tissue sample to be investigated. The number of windings should be as high as possible given the chosen geometry. However, if the excitation coil 46 and receiver coil 48 are provided on a printed circuit board (PCB), the number of windings can be as low as 1 or 2. However, the strength of the excitation field is linearly proportional to the number of windings and the current, so more windings could be beneficial. An exemplary diameter for the excitation coil 46 and receiver coil 48 is 5 cm, although other diameters can be used.

The control unit 50 comprises excitation coil driving circuitry 52 that generates an alternating current having magnitude I and frequency f for driving the excitation coil 46 to generate a magnetic field, and a processing unit 54 that receives the voltage signal induced in the receiver coil 48 by the eddy current in the tissue sample 44 and determines an indication of the tissue fluid content (e.g. the conductivity of the tissue sample 44 or the phase difference $\Delta_\varphi$) from the voltage signal and a reference signal from the excitation coil driving circuitry 52 that corresponds to the voltage signal used to drive the excitation coil 46. The frequency of the alternating current from the driving circuitry 52 is typically in the range of 1-10 MHz

The apparatus 42 also comprises a second excitation coil 56 and a reference coil 58. The second excitation coil 56 and the reference coil 58 preferably correspond to the first excitation coil 46 and receiver coil 48 respectively in construction and relative arrangement (i.e. in the illustrated embodiment the second excitation coil 56 and reference coil 58 are in the same orientation with respect to each other - their axes are parallel) so that the second excitation coil 56 and the reference coil 58 respond in a similar way to the first excitation coil 46 and receiver coil 48. If the second excitation coil 56 and the reference coil 58 do not correspond to the first excitation coil 46 and receiver coil 48 respectively, the difference in response will need to be determined from calibration experiments. The second excitation coil 56 is connected to the excitation coil driving circuitry 52 and is driven using the same alternating current as the first excitation coil 46. The reference coil 58 is arranged close to the second excitation coil 56 so that the magnetic field generated by the second excitation coil 56 induces a current in the reference coil 58. Since the signals in the reference coil 58 and second excitation coil 56 are of similar amplitude, the reference coil 58 is connected to the processing unit 54 and the receiver coil 48 in a gradiometer-like way (i.e. the reference coil 58 and receiver coil 48 are connected in series such that the direction of the currents in the receiver coil 48 and reference coil 58 are opposite to each other, i.e. flow in opposite directions). This connection arrangement is shown as shown in Figure 4. When no tissue sample is near to the receiver coil 48 and the apparatus 42 is operated, the signal in the receiver coil 48 and the signal in the reference coil 58 will be exactly 180° out of phase with each other which means they will cancel each other out at each point in time. When a tissue sample is near to the receiver coil 48, a phase delay will be introduced to the signal in the receiver coil 48 which will cause the signal to be out of phase with the signal induced in the reference coil 58, and the resulting signal from the interconnected coils 48, 58 will represent the signal of interest, i.e. the voltage induced by the eddy current in the tissue sample 44.

Thus, connecting the reference coil 58 and receiver coil 48 in this way causes subtraction of the signal in the reference coil 58 from the signal in the receiver coil 48 in the analog domain (i.e. before any analog-to-digital (A/D) conversion), since the connection between the coils 48, 58 means the induced currents are flowing in opposite directions, which just leaves the signal of interest, namely the signal in the receiver coil 48 representing the current induced by the eddy current in the tissue sample 44. This is much simpler than in the arrangement of Figure 2(c) where the amplitude of the signal in the reference coil is much greater than the signal in the receiver coil which means that the subtraction of the signals has to be done in the digital domain (i.e. after analog-to-digital conversion), which significantly limits the number of bits available to find the phase difference $\Delta_\varphi$. Effective subtraction in the analog domain with the arrangement in Figure 3 leaves the whole bit range of an A/D converter to find the phase difference $\Delta_\varphi$. Another advantage of the embodiment in Figure 3 is that the second excitation coil 56 and the reference coil 58 do not have to be immediately adjacent to the tissue sample 44 or the other coils 46, 48, as long as they are in the same environment (i.e. same temperature, humidity, etc).

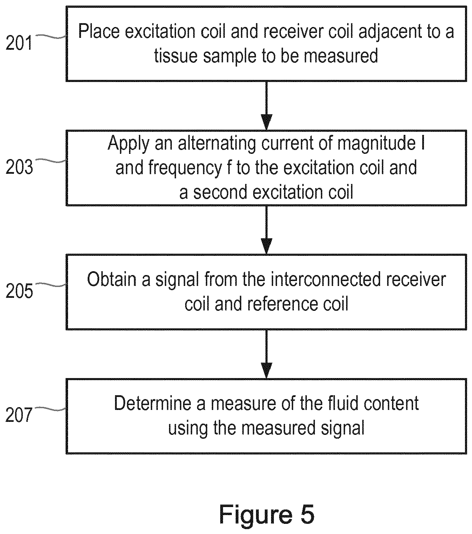

A method of using magnetic induction spectroscopy to monitor tissue fluid content according to the invention is illustrated by the flow chart in Figure 5.

In a first step, the MIS apparatus 42 is placed adjacent the tissue sample 44 that is to be measured (step 201). That is, the first excitation coil 46 and receiver coil 48 are placed adjacent to the tissue sample 44 to be measured.

In step 203, the excitation coil driving circuitry 52 is operated to generate an alternating current of magnitude I and frequency f that is supplied to the first excitation coil 46 and the second excitation coil 56 in order for those coils to generate magnetic fields.

A (voltage) signal is induced in the receiver coil 48 by the magnetic field generated by the first excitation coil 46 and by the magnetic field generated by the eddy current in the tissue sample 44, and a (voltage) signal is induced in the reference coil 58 by the magnetic field generated by the second excitation coil 56. By connecting the reference coil 58 and receiver coil 48 in series such that the currents induced therein flow in opposite directions, the processing unit 54 obtains a signal from the coils 48, 58 that represents the signal induced in the receiver coil 48 by the eddy current in the tissue sample 54 (step 205). Thus, connecting the reference coil 58 and receiver coil 48 in this way and providing a second excitation coil 56 allows the effects of coil crosstalk between the first excitation coil 46 and the receiver coil 48 to be removed.

In step 207, the processing unit 54 determines a measure of the fluid content of the tissue sample 44 using the signal from the coils 48, 58. The measure of the fluid content can be an indication of the conductivity of the tissue sample 44.

Preferably, the signal obtained from the reference coil 58 and receiver coil 48 (which comprises the current induced by the eddy current in the tissue sample 44, the current induced in the receiver coil 48 by the magnetic field from the first excitation coil 46 and the current induced in the reference coil 58 by the magnetic field from the second excitation coil 56, the latter of which is effectively cancelled out by the current induced in the receiver coil 48 by the magnetic field from the first excitation coil 46 due to the gradiometer-like connection) is amplified and demodulated by analog electronics in the processing unit 54. After amplification and demodulation the signal is converted to the digital domain using an analog to digital converter in the processing unit 54. Once the signal is in the digital domain, the real and imaginary parts of the received signal can be obtained. A signal ratio (received signal/excitation field) can be expressed by the following formula

$$\frac{\Delta V}{V} = Q\omega\mu_0[\omega\varepsilon_0(\varepsilon_r - 1) - i\sigma] + R(\mu_r - 1) \tag{1}$$

Where, in the embodiment of Figure 3, $\Delta V$ = ($V_{reference}$ + $V_{receiver}$), with $V_{receiver}$ being the induced voltage in the receiver coil from the eddy current in the tissue sample and $V_{reference}$ being the primary field (the induced voltage obtained by the reference coil), respectively. V is the reference signal corresponding to the voltage signal from the excitation coil driving circuitry 52 that is used to drive the excitation coils 46, 56. The electrical conductivity (the remaining variable in Equation 1 once $\Delta V$ and V are known), the relative permittivity and the relative permeability of the sample are represented by $\sigma$, $\varepsilon_r$ and $\mu_r$ respectively. The permittivity and permeability are $\varepsilon_0$ and $\mu_0$, respectively. The geometrical values $Q$ and $R$ are related to the coil configuration. The imaginary component of the signal ratio in Equation 1 ($\omega\varepsilon_0$ -

($\varepsilon_r$- 1)) is proportional to the tissue conductivity. The real part of the signal ratio is (i$\sigma$). Once the phase angle is known, the tissue conductivity can be found by taking the arctan of the phase angle. The processing of the signal from the coils 48, 58 and the reference signal (which provides the ratio $\Delta V/V$ on the left hand side of Equation 1) in step 207 to determine the measure of the fluid content of the tissue sample 44 is generally conventional and will be understood by those skilled in the art.

It will be appreciated that the MIS apparatus 42 in Figure 3 is thus more sensitive to changes in the fluid content of the tissue sample 44 than conventional MIS apparatus, and is stable in the presence of temperature and humidity fluctuations and less sensitive to movement of the apparatus and/or patient during measurements.

As noted above, a significant drawback of using MIS for biomedical purposes is that it is sensitive to the relative movement between coils and relative movement between the coils and the tissue sample. Furthermore, since MIS only examines the tissue sample in the excitation magnetic field, tissue inhomogeneities (e.g., bones and large blood vessels) also affect the measurement. On the one hand, this is a strength of MIS as it allows the detection of local tissue characteristics (e.g., brain edema) but on the other hand, it is a weakness when one wants to apply MIS to measure 'global' (i.e., not site-specific) under- or over-hydration.

Although the apparatus in Figure 3 mitigates the above drawbacks to some extent, further improvements in the robustness of the MIS apparatus against relative movements between the coils and/or between the coils and the tissue sample can be obtained through a modification to the apparatus according to the first embodiment shown in Figure 3.

In particular, a solution to the movement problem is to configure the first excitation coil 46 so that it can be placed around the tissue sample 44 to be measured. That is, the excitation coil is placed around the tissue sample 44 so that the tissue sample is in the centre (core) of the coil 46. When applied to the MIS apparatus 42 shown in Figure 3, the receiver coil 48 is also configured such that it can be placed around the tissue sample 44 to be measured. Thus the coils 46, 48 are configured so that they can be placed around a finger, toe, wrist, ankle, arm, leg, waist, torso of the patient as required for the measurement to be taken. The coils 46, 48 can thus be arranged in an item of clothing or jewellery (e.g. a ring, bracelet or watch) so that they can be easily and consistently used by the patient to measure the fluid content of the tissue sample 44. By placing the excitation coil 46 and receiver coil 48 around the tissue sample 44 to be measured, the range of movement of the coils 46, 48 relative to the tissue sample 44 is limited by the diameter of the coils 46, 48 relative to the diameter of the tissue sample 44.

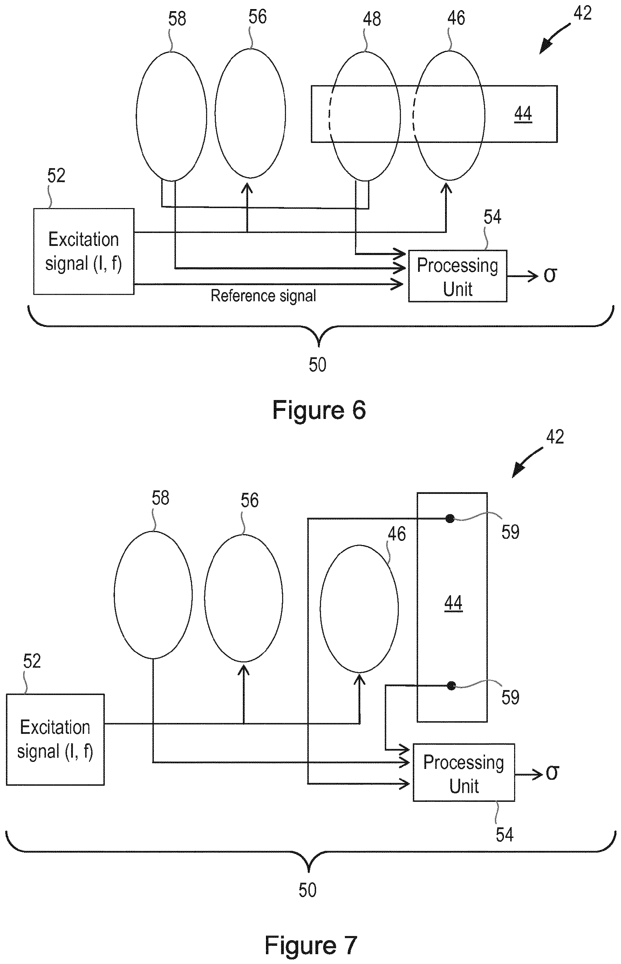

A MIS apparatus 42 according to a second embodiment is shown in Figure 6, where it can be seen that the first excitation coil 46 and receiver coil 48 are placed around the tissue sample 44. As the first excitation coil 46 is placed around the tissue sample 44, the magnetic field from the excitation coil 46 does not need to be as strong as in the embodiment of Figure 3 in order to generate a sufficiently strong eddy current in the tissue sample 44 for it to be detected by the receiver coil 48. As well as being more robust against relative movement of the apparatus 42 and the tissue sample 44, this embodiment also requires less power in order to make the measurements, which makes it more suitable for portable implementations (e.g. a device that the patient wears continuously). Other than the configuration of the excitation coil 46 and receiver coil 48, the MIS apparatus 42 according to the second embodiment operates in the same way as the apparatus 42 in the first embodiment (i.e. with the first excitation coil 46 and second excitation coil 56 being driven with a common driving signal).

A first example of a MIS apparatus according to a first alternative implementation is shown in Figure 7. In this first example, to avoid problems with coil crosstalk between the excitation coil and receiver coil, the receiver coil is replaced by two or more (e.g. four) electrodes that are placed on the skin of the patient around the measurement site and that measure the potential (voltage) created by the eddy current induced in the tissue sample by the magnetic field from the excitation coil.

The advantage of this first example over BIS (which uses skin-mounted electrodes) is that MIS allows the tissue in a fixed volume (i.e. within the magnetic field) to be examined, whereas the measurement volume of BIS is not fixed since the distribution of the current depends on the path of least resistance. This has the implication that a MIS apparatus according to this first example still allows control over the measurement depth even though it is using skin-mounted electrodes.

The MIS apparatus 42 according to the first example shown in Figure 7 corresponds generally to the first embodiment, with like components having the same reference numerals. However, the receiver coil 48 has been replaced by two electrodes 59 that are for attachment to the skin of the tissue sample 44 around the volume to which the magnetic field from the excitation coil 46 is applied. It will also be noted that the reference coil 58 is now directly connected to the processing unit 54.

In operation, the first excitation coil 46 and second excitation coil 56 are driven with an alternating current to generate respective magnetic fields (as in the embodiments of Figure 5 and 6 the same driving signal is provided to each coil 46, 56). The processing unit 54 receives a signal from the reference coil 58 representing the current induced in the coil 58 by the magnetic field from the second excitation coil 56 and a voltage signal from the electrodes 59 representing the eddy current induced in the tissue sample 44 by the magnetic field from the first excitation coil 46. The processing unit 54 generally processes these signals in a similar way to the signal obtained in the embodiment of Figure 3 above and shown in step 207 of Figure 5 (although in this embodiment the ratio ∆V/V in Equation 1 is determined by dividing the signal from the electrodes 59 by the signal from the reference coil 58). However, the difference compared to the embodiment of Figure 3 is that there needs to be a phase adjustment of the received signal from the electrodes 59 before comparison with the signal from the reference coil 58 (due to electrode measurement principle). The comparison of the phase-adjusted received signal from the electrodes 59 with the signal from the reference coil 58 comprises subtracting the signal from the reference coil 58 from the phase-adjusted received signal.

The amount of the phase adjustment can be determined in advance of an actual MIS measurement during a reference or calibration experiment in which no tissue sample is present and the phase delay between the signal from the electrodes 59 and the signal from the reference coil 58 is measured. Once this baseline phase delay is known, this should be corrected for to only account for phase delay caused by the tissue sample.

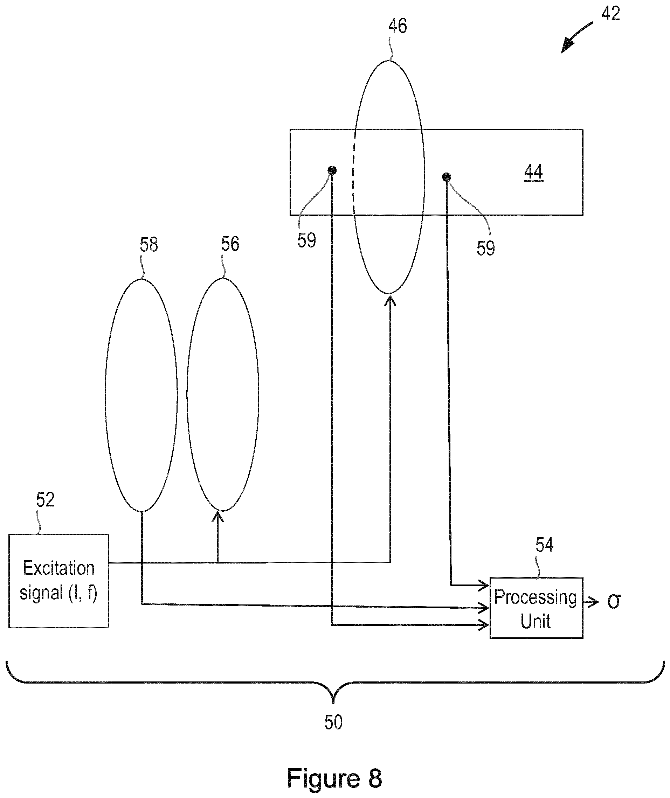

A second example of a MIS apparatus according to the first alternative implementation is shown in Figure 8. This MIS apparatus 42 represents a combination of the second embodiment and the first example of the first alternative implementation described above, and thus provides a first excitation coil 46 that is configured to be placed around the tissue sample 44 to be measured and comprises two or more electrodes 59 for attachment to the skin of the patient in place of a receiver coil 48. This second example operates in generally the same way as the first example described above, but having the first excitation coil 46 around the tissue sample 44 enables less power to be used to generate the magnetic fields from the first excitation coil 46 and second excitation coil 56.

In addition to the above invention and first alternative implementation, a second alternative implemention is provided in which the improvement of placing an excitation coil around the tissue sample to be measured is applied to the conventional MIS arrangements shown in Figures 2(a), (b) and (c). As in the second embodiment of the invention above, placing the excitation coil around the tissue sample to be measured reduces or prevents relative movement between the coils and the tissue sample.

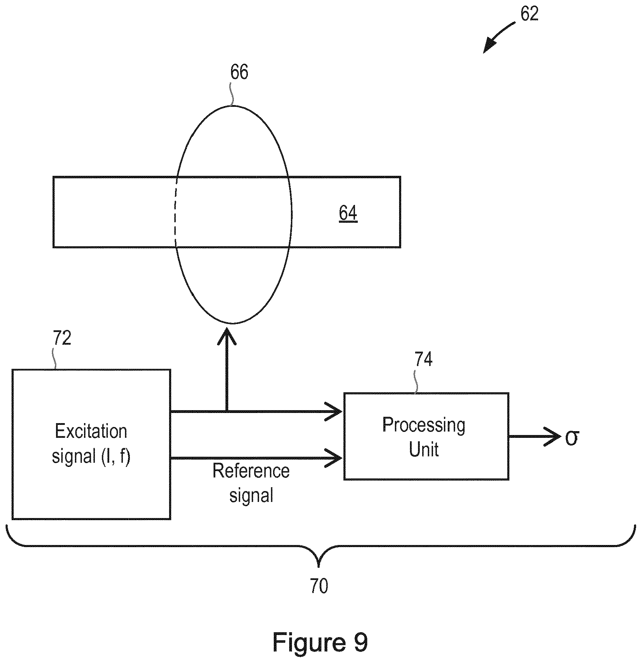

A first example of the second alternative implemention is shown in Figure 9. The MIS apparatus 62 in this figure corresponds to the arrangement shown in Figure 2(a) with the excitation coil placed around the tissue sample 64.

The MIS apparatus 62 comprises an excitation coil 66 that is configured so that it can be placed around a finger, toe, wrist, ankle, arm, leg, waist, torso of the patient as required for the measurement to be taken. The coil 66 can thus be arranged in an item of clothing or jewellery (e.g. a ring, bracelet or watch) so that they can be easily and consistently used by the patient to measure the fluid content of the tissue sample 64.

The excitation coil 66 is connected to a control unit 70 that comprises excitation coil driving circuitry 72 that generates an alternating current having magnitude I and frequency f for driving the excitation coil 66 to generate a magnetic field, and a processing unit 74 that measures the impedance change in the excitation coil 66 caused by the eddy current in the tissue sample 64, receives a reference signal from the excitation coil driving circuitry 72 corresponding to the signal used to drive the excitation coil 66, and determines an indication of the tissue fluid content (e.g. a measure of the conductivity of the tissue sample 64).

The inductance of the excitation coil 66 will reduce when the conductivity of the tissue sample 64 increases due to increased energy transfer to the tissue sample 64 in the form of eddy currents. With the excitation coil adjacent to the tissue sample as in 'conventional' MIS in Figure 2(a), the driving signal of the excitation coil must be powerful enough to generate sufficient eddy currents in the tissue sample to create, in turn, a sufficiently large eddy current-induced magnetic field. However, such a strong excitation field makes it difficult, if not impossible, to detect the eddy current-induced magnetic field with the same coil, so a separate receiver coil is needed. However, this introduces the problem of coil crosstalk. Designing the excitation coil such that it would be sensitive to the magnetic field created by the eddy currents in the tissue sample, would limit the penetration depth of the technique dramatically. In contrast, when placing the excitation coil 66 around the tissue sample, the excitation coil 66 can be designed (e.g. in terms of diameter of wire, number of windings, etc.) such that the effect of the eddy currents in the tissue sample can be measured with the same coil (so no separate receiver coil is required). In some examples, the diameter of the wire can be 0.5-1mm and there can be 2-4 windings in the coil.

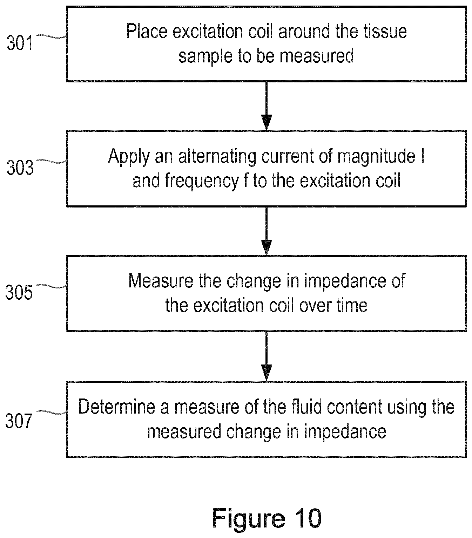

A method of using magnetic induction spectroscopy to monitor tissue fluid content according to the second alternative implementation is illustrated by the flow chart in Figure 10.

In a first step, the excitation coil 66 of the MIS apparatus 62 is placed around the tissue sample 64 that is to be measured (step 301).

In step 303, the excitation coil driving circuitry 72 is operated to generate an alternating current of magnitude I and frequency f that is supplied to the excitation coil 66 in order for the coil to generate a magnetic field in the tissue sample 64.

The magnetic field generated by the excitation coil 66 causes an eddy current in the tissue sample 64 which itself causes a change in impedance in the excitation coil 66. This change in impedance of the excitation coil 66 is measured over time by the processing unit 74 (step 305).

In step 307, the processing unit 74 determines a measure of the fluid content of the tissue sample 64 using the measured change in impedance. Preferably the processing unit 74 determines the difference between the signal in the excitation coil 66 and the reference signal to obtain the tissue signal (this is equal to ∆V in equation (1) above). The properties of the tissue can be determined in the same way as is described above for step 207.

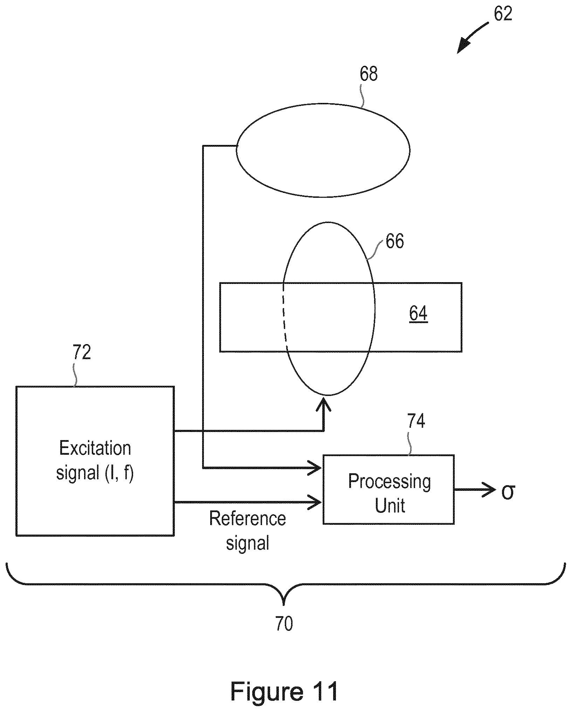

A second example of the second alternative implementation is shown in Figure 11. The MIS apparatus 62 in this figure corresponds to the arrangement shown in Figure 2(b) but with the excitation coil placed around the tissue sample 64. Thus in this example, the excitation coil 66 is placed around the tissue sample 64 and a receiver coil 68 is provided adjacent to the tissue sample 64 and arranged orthogonally to the excitation coil 66 to minimise the crosstalk between the coils.

As in the arrangement of Figure 2(b), the processing unit 74 performs a phase measurement between the excitation coil 66 and the receiver coil 68 to determine the indication of the tissue fluid content. The processing performed by the processing unit 74 to determine the indication of the tissue fluid content can be the same as that performed in the conventional apparatus of Figure 2(b), and thus will not be described in detail herein.

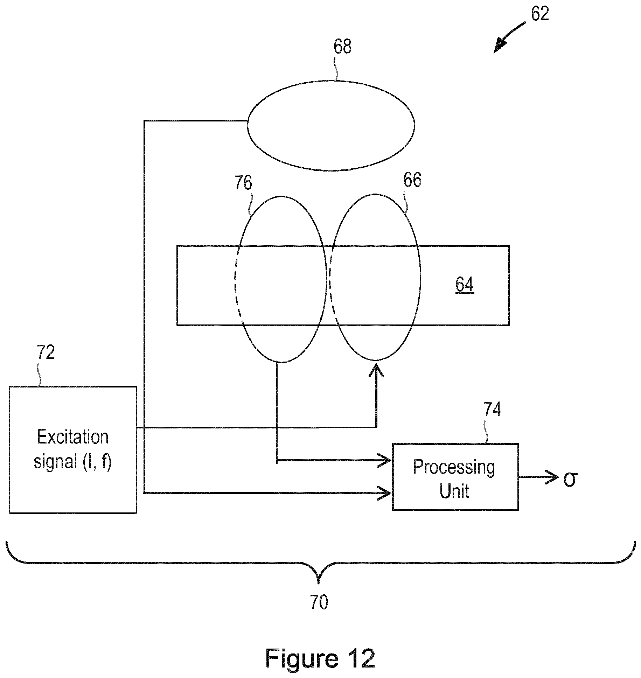

A third example of the second alternative implemention is shown in Figure 12. The MIS apparatus 62 in this figure corresponds to the arrangement shown in Figure 2(c) but with the excitation coil placed around the tissue sample 64. Thus in this example, the excitation coil 66 and a reference coil 76 is placed around the tissue sample 64, and a receiver coil 68 is provided adjacent to the tissue sample 64 that is arranged orthogonally to the excitation coil 66 to minimise the crosstalk between the coils.

As in the arrangement of Figure 2(c), the processing unit 74 measures the phase difference ∆φ between the receiver coil 68 and the reference coil 76 (which has a strong mutual induction with the excitation coil 66) to determine an indication of the tissue fluid content. The processing performed by the processing unit 74 to determine the indication of the tissue fluid content can be the same as that performed in the conventional apparatus of Figure 2(c), and thus will not be described in detail herein.

The example shown in Figure 12 provides all of the advantages of the arrangement of Figure 2(c), i.e. that the effects of variations in the excitation coil driving circuitry and excitation coil, e.g. due to temperature variations, are strongly suppressed, along with some compensation of the drift in the detection system by matching the receiver coil 68 and reference coil 76, and also has the advantage provided by arranging the excitation coil 66 around the tissue sample to be measured that movements of the apparatus relative to the patient are reduced.

In addition to the above invention and the first and second alternative implementions, a third alternative implemention is provided in which the improvement of using two or more electrodes to measure the potential (voltage) resulting from the induced eddy current in the tissue sample is applied to the conventional MIS arrangements shown in Figures 2(b) and (c). As in the first alternative implementation above, this aspect avoids problems with coil crosstalk between the excitation coil and receiver coil.

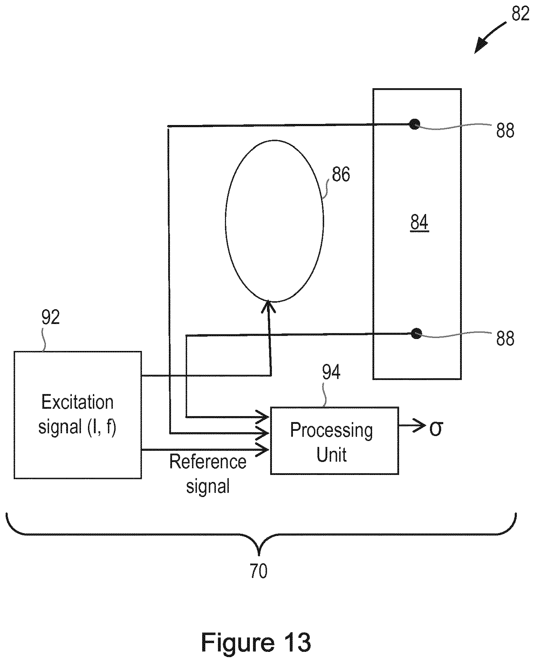

A first example of the third alternative implementation is shown in Figure 13. The MIS apparatus 82 in this figure corresponds to the arrangement shown in Figure 2(b) with the receiver coil replaced by a pair of electrodes at the measurement site on the tissue sample 84.

The MIS apparatus 82 comprises an excitation coil 86 that is placed adjacent to the tissue sample 84 to be measured. The excitation coil 86 is connected to a control unit 90 that comprises excitation coil driving circuitry 92 that generates an alternating current having magnitude I and frequency f for driving the excitation coil 86 to generate a magnetic field, and a processing unit 94 that measures the potential between the electrodes 88, receives a reference signal from the excitation coil driving circuitry 92 corresponding to the signal used to drive the excitation coil, and determines an indication of the tissue fluid content (e.g. a measure of the conductivity of the tissue sample 84).

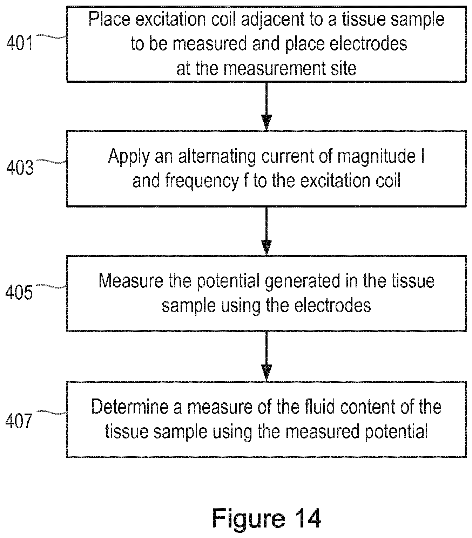

A method of using magnetic induction spectroscopy to monitor tissue fluid content according to the third alternative implementation is illustrated by the flow chart in Figure 14.

In a first step, the excitation coil 86 is placed adjacent the tissue sample 64 that is to be measured and the electrodes 88 are attached around the area of the tissue sample 84 to be measured (step 401).

In step 403, the excitation coil driving circuitry 92 is operated to generate an alternating current of magnitude I and frequency f that is supplied to the excitation coil 86 in order for the coil to generate a magnetic field in the tissue sample 84.

The magnetic field generated by the excitation coil 86 causes an eddy current in the tissue sample 84 which is measured by the electrodes 88 attached to the tissue sample 84 (step 405).

In step 407, the processing unit 94 determines a measure of the fluid content of the tissue sample 84 using the signal from the electrodes 88.

Preferably in step 407 the processing unit 94 performs a phase adjustment of the received signal from the electrodes 88 before comparison with the reference signal from the excitation coil driving circuitry 92. This phase adjustment is similar to that described above for the example in Figure 7. The comparison of the phase-adjusted received signal from the electrodes 88 with the reference signal from the excitation coil driving circuitry 92 comprises subtracting the reference signal from the phase-adjusted received signal. The resulting signal is then processed by the processing unit 94 as described above for step 207 (e.g. the signal is amplified and demodulated, converted to the digital domain, the real and imaginary parts are obtained from the signal ratio (received signal/excitation field)).

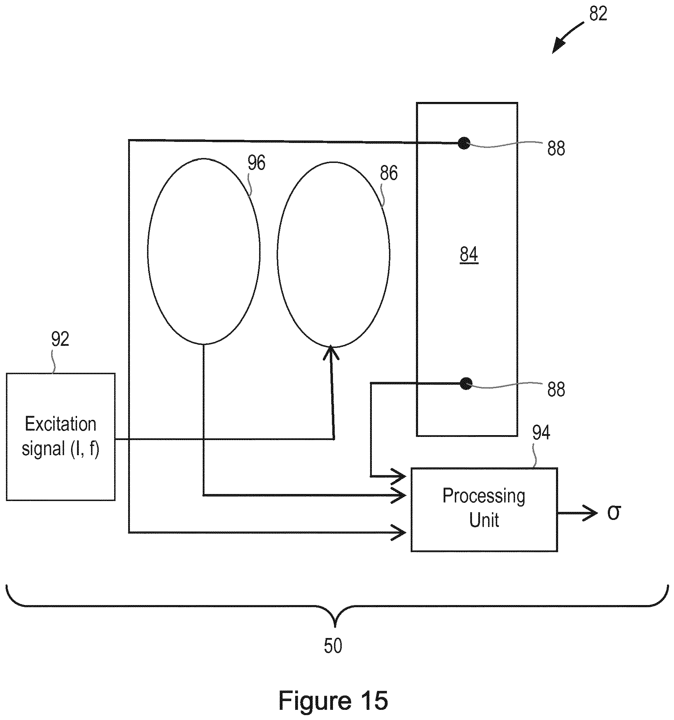

A second example of the third alternative implementation is shown in Figure 15. The MIS apparatus 82 in this figure corresponds to the arrangement shown in Figure 2(c) but with the receiver coil replaced by electrodes 88 that are in contact with the tissue sample 84. Thus in this example, the excitation coil 86 is placed adjacent to the tissue sample and a reference coil 96 is placed near to the excitation coil 86.

The processing unit 94 measures the phase difference Δφ between the potential measured by the electrodes 88 and the reference coil 96 (which has a strong mutual induction with the excitation coil 86) to determine an indication of the tissue fluid content. Preferably the processing unit 94 applies a phase adjustment to the received signal from the electrodes 88 before comparison with the signal from the reference coil 96. This phase adjustment is similar to that described above for the example in Figure 7. The comparison of the phase-adjusted received signal from the electrodes 88 with the reference signal from the reference coil 96 comprises subtracting the reference signal from the phase-adjusted received signal. The resulting signal is then processed by the processing unit 94 as described above for step 207 (e.g. the signal is amplified and demodulated, converted to the digital domain, the real and imaginary parts are obtained from the signal ratio (received signal/excitation field)).

The example shown in Figure 15 provides the advantages of the arrangement of Figure 2(c), i.e. that the effects of variations in the excitation coil driving circuitry and excitation coil, e.g. due to temperature variations, are strongly suppressed, and also has the advantage that there is no crosstalk between the excitation coil 86 and a receiver coil.

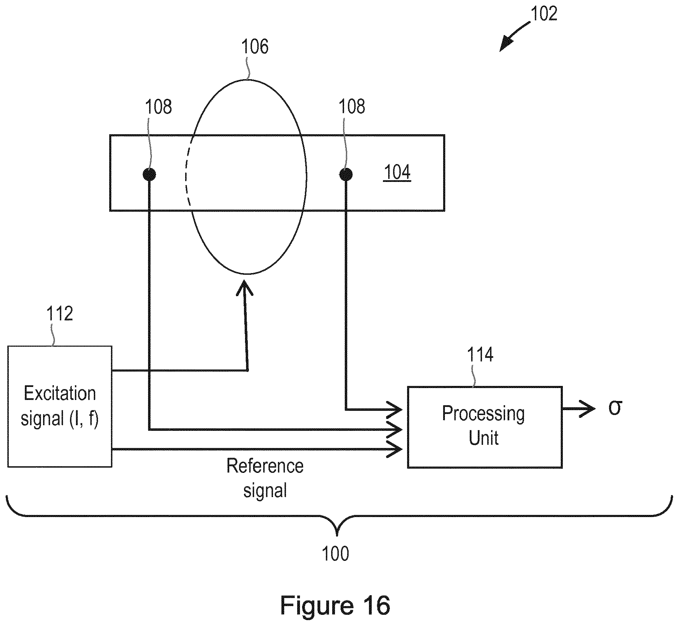

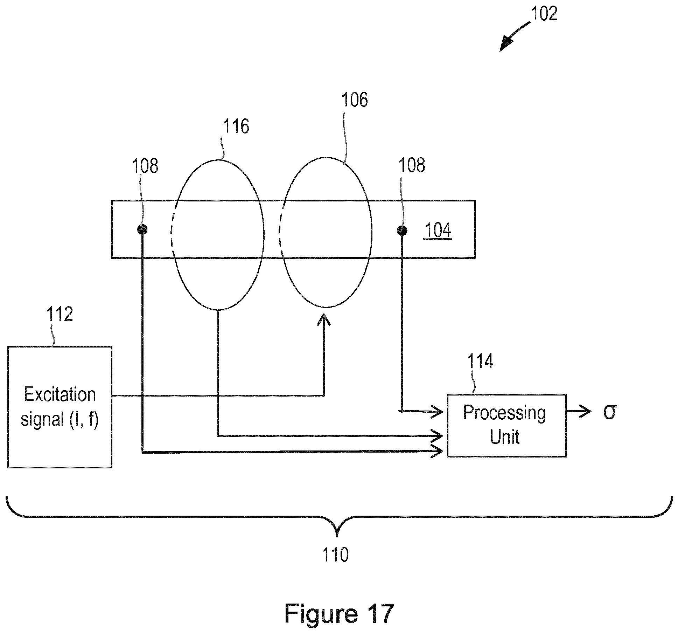

It will be appreciated that the improvements provided by the second and third alternative implementations can also be used together in a MIS apparatus to reduce the motion sensitivity of the apparatus and avoid the problems with crosstalk between the excitation coil and the receiver coil. Figures 16 and 17 illustrate two examples of a MIS apparatus 102 in which the excitation coil is placed around the tissue sample to be measured and two or more electrodes are used to measure the potential of the eddy current.

The MIS apparatus 102 in Figure 16 is based on the arrangement in Figure 2(b). The MIS apparatus 102 is for measuring the fluid content of a tissue sample 104 and comprises an excitation coil 106 that is configured to be placed around the tissue sample 104 to be measured. Two or more electrodes 108 are connected or attached to the tissue sample 104 for measuring the potential in the tissue sample. The excitation coil 106 and electrodes 108 are connected to a control unit 110 that comprises excitation coil driving circuitry 112 that generates an alternating current having magnitude I and frequency f for driving the excitation coil 106 to generate a magnetic field, and a processing unit 114 that measures the potential between the electrodes 108, receives a reference signal from the excitation coil driving circuitry 112 corresponding to the signal used to drive the excitation coil, and determines an indication of the tissue fluid content (e.g. a measure of the conductivity of the tissue sample 104). The processing performed by processing unit 114 is the same as described above for the example of Figure 13.

The MIS apparatus 102 in Figure 17 is based on the arrangement in Figure 2(c). The MIS apparatus 102 comprises excitation coil 106 that is configured to be placed around the tissue sample 104 to be measured and two or more electrodes 108 are connected or attached to the tissue sample 104 for measuring the potential in the tissue sample. A reference coil 116 is also provided that is configured to be placed around the tissue sample 104. The excitation coil 106, reference coil 116 and electrodes 108 are connected to control unit 110 which determines an indication of the tissue fluid content (e.g. a measure of the conductivity of the tissue sample 104) from the signal from the reference coil 116 and the measured potential between the electrodes 108. The processing performed by processing unit 114 is the same as described above for the example of Figure 15.

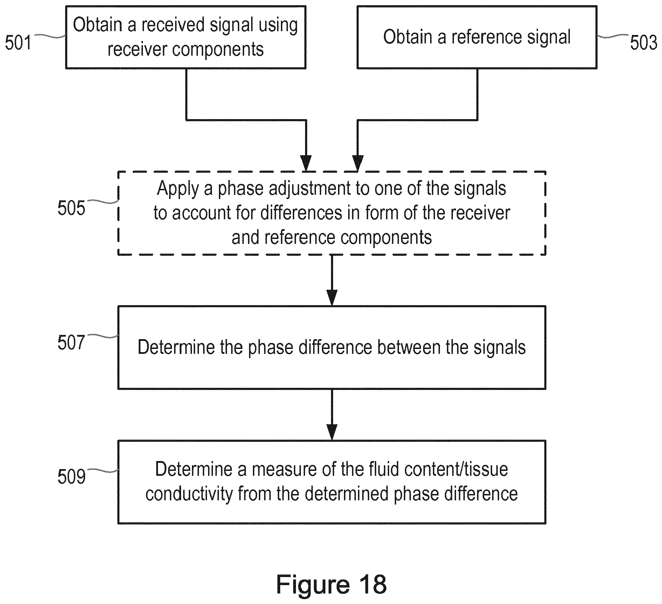

A general method of using magnetic induction spectroscopy, MIS, to determine a measure of the fluid content of a tissue sample is shown in the flow chart of Figure 18. This method is applicable to all of the above embodiments and examples.

Thus, an excitation coil in the MIS apparatus is driven with an alternating current in order to cause the excitation coil to generate a magnetic field and thus an eddy current in a tissue sample. This eddy current generates a magnetic field and thus a current in a receiver component in the MIS apparatus. This current is measured by the receiver component (step 501 of Figure 18). Due to the tissue sample, this measured current (received signal) will have a phase delay (and small amplitude reduction) compared to the signal used to drive the excitation coil. Assuming the excitation signal is a sine wave, the received signal will also be a sine wave (but phase-delayed with respect to the excitation signal).

It will be noted from the above that in the example of Figure 9, the receiver component that obtains the signal in step 501 is the excitation coil itself, whereas in the embodiments of Figures 3 and 6 and the examples of Figures 11 and 12 the receiver component is a receiver coil that is arranged near to the tissue sample and in the examples of Figures 7, 8, 13, 15, 16 and 17 the receiver component are electrodes that are placed on the tissue sample.

A reference signal is also required in order to determine the phase difference induced in the received signal by the eddy current in the tissue sample. Thus, a reference signal is obtained from a reference signal component (step 503). Assuming the excitation signal is a sine wave, the reference signal will also be a sine wave.

In some embodiments (in particular the embodiments of Figures 3 and 6 and the examples of Figures 9, 11, 13 and 16), the reference signal is the electrical signal used to drive the excitation coil to generate the magnetic field, and thus the reference signal component is the excitation coil driving circuitry. It will be noted that in the embodiments of Figures 3 and 6, the signal from the reference coil is coupled to the signal from the receiver coil in order to produce a signal that just represents the current induced in the receiver coil by the eddy current in the tissue sample. In the alternative implementations, (in particular the examples of Figures 7 and 8) the reference signal is the signal obtained from a reference coil that is placed near to a second excitation coil (that is driven with the same signal as the first excitation coil) and thus the reference signal component is the second excitation coil and the reference coil. In the remaining examples (the examples of Figures 12, 15 and 17) the reference signal is the signal obtained from a reference coil that is placed near to the excitation coil, and thus the reference signal component is the reference coil.

After obtaining the received signal and reference signal, in some embodiments/examples it is necessary to apply a phase adjustment to the received signal (although it will be appreciated that the opposite adjustment could instead be applied to the reference signal) to account for differences in the components used to obtain the received signal and reference signal (in particular differences introduced through the use of electrodes to obtain the received signal). Thus, in step 505, which is applicable to the examples that use electrodes to obtain the received signal (the examples of Figures 7, 8, 13, 15, 16 and 17), a phase adjustment is applied to one of the signals. As described above, the amount of the phase adjustment can be determined in a calibration experiment when no tissue sample is present.

Next, in step 507, the phase difference between the received signal (phase-adjusted, if appropriate) and the reference signal is determined. In all examples (so excluding the embodiments in Figures 3 and 6), step 507 comprises subtracting one signal from the other (in the digital domain) to give the phase difference. In these examples, as most of the bit range of the A/D converter (used to convert the analog signals to the digital domain) is dedicated to describe the large signals of the excitation electronics, the effects of the tissue magnetic field are very small compared to these signals and only covers a few bits.

In the embodiments of Figures 3 and 6, the phase difference is obtained in step 507 through connecting the receiver coil and reference coil such that the currents induced therein flow in opposite directions to create a gradiometer and comparing the resulting signal to the reference signal. When neither coil is around or adjacent to tissue, the reference signal and receiver signal are exactly 180 degrees out of phase and will cancel each other out at each point in time. However, when the receiver coil is adjacent or around tissue, a phase delay is introduced in the receiver signal, which causes the signal to be out of phase with the reference signal, so the signal obtained from the receiver and reference components is a signal that is the sum of the signal in the receiver coil and the signal in the reference coil, which represents the signal of interest; i.e. the signal coming from the tissue. In these embodiments, the full bit range of the A/D converter (used to convert the signal from the interconnected receiver coil and reference coil to the digital domain) is dedicated to describe the tissue signal, which yields a much better resolution than the other embodiments.

Once the phase delay has been determined in step 507, the phase delay is used to determine a measure of the tissue conductivity (and thus water content) of the tissue sample (step 509). In preferred implementations, the tissue conductivity is determined using a calibration function or table that maps values of phase delay to tissue conductivity.

Further variations of the above-described embodiments and examples are contemplated to further improve the reliability and versatility of MIS for measuring tissue fluid content. Some of these variations are described below.

Multi-depth and differential-depth MIS - The measurement depth of MIS (i.e. the extent of the measurement volume in the tissue sample) depends on the strength of the excitation field (which is dependent on the current through the excitation coil) and the radius of the excitation coil. Increasing the strength of the excitation field (by increasing the magnitude of the alternating current) increases the depth of the measurement and vice versa.

Therefore, in some embodiments and examples the strength of the excitation field can be set at a particular value to effect a MIS measurement at a particular depth (e.g. to measure the fluid content of a particular subsurface tissue such as the lungs or muscles). In other embodiments and examples, multiple MIS measurements can be performed with different strength excitation fields to obtain measurements of the fluid content at different depths in the tissue sample. These measurements can be combined to obtain differential-depth measurements (i.e. which indicate the differences in fluid content between different depths). Differential-depth measurements are more robust against movement artefacts than single depth MIS measurements.

Use of heart beat-induced modulation of the MIS signal - As suggested above, as well as being sensitive to the long-term amount of fluid in a tissue sample, the MIS measurements obtained using the above aspects and embodiments are also sensitive to the heart beat-induced pulse pressure waves since these pressure waves temporarily change the composition of the tissue in the MIS excitation field (i.e. due to an increase in blood volume). Therefore, taking a number of MIS measurements in a short interval enables MIS measurements to be used for the simultaneous assessment of heart rate.

Further, ventilation-related changes in intra-thoracic pressure and cardiac preload lead to variations in stroke volume and therefore also to variations in the amplitudes of the pulse pressure waves. Detecting this ventilation-induced pulse pressure modulation in the MIS measurements allows for the respiration rate to be assessed. These pulse pressure variations (PPV), moreover, are considered a measure of a patient's volume status; high PPV have been associated with hypovolemia and fluid responsiveness. The measurement of heart rate, ventilation rate, and PPV is possible with all of the embodiments and examples described herein.

Use of accelerometers to time MIS measurements - Even with the above embodiments and examples, the MIS measurements are still sensitive to relative movement between the coils themselves and relative movement between the coils and the tissue sample. Therefore, in some embodiments and examples, a motion sensor, such as an accelerometer, can be included in the MIS apparatus (and preferably attached to the patient close to the measurement site (tissue sample)), and the signal from the motion sensor processed to determine the amount of movement (e.g. a magnitude, intensity, etc.) and to determine if the patient is moving too much through comparison of the amount of movement with a threshold value (e.g. if the magnitude or intensity of acceleration measured by the accelerometer is greater than a threshold value at a given time instant or at any point during a given time period then the amount of movement can be considered too high). In some cases a MIS measurement can be discarded if it is determined that there is too much motion, and in other cases the MIS apparatus can be controlled so that it does not perform a MIS measurement until the level of motion decreases to a suitable level (e.g. below the threshold).

APPLICATIONS OF THE INVENTION

The MIS apparatus described above can provide feedback on a patient's hydration status, vital signs such as heart rate, and provide a warning of under- or over-hydration to either the patient themselves (e.g. through some direct indicator that is part of the apparatus) or to medical personnel (e.g. through communicating wirelessly, such as with Wi-Fi, Bluetooth or a cellular telecommunication technology to a remote location). Since MIS in principle does not require tissue contact, it is possible to implement the apparatus in a number of different forms. For example the MIS apparatus could be formed as an anklet, bracelet, watch, ring, textile, or a hand-held device.

Early warning for hypovolemia - Hypovolemia is common in various very large patient populations in the ward, intensive care unit (ICU), and operating room (OR) due to: insufficient oral/intravenous fluid intake, vasorelaxing anaesthesia, internal bleeding, sepsis, kidney dysfunction, and increased fluid losses due to mechanical ventilation, fever, vomiting, and diarrhoea. Mild states of hypovolemia before surgery have been associated with worse outcomes. A hypovolemia-check could reveal occult internal bleeding in patients in the ambulance and in the emergency room (ER). Since the first compensation mechanism for correction of hypovolemia is shifting fluids from the tissue to the blood, tissue (de)hydration and patient volume status are physiologically related. A MIS apparatus measuring (a loss of) tissue water would therefore provide an early warning for hypovolemia in hospitalised patients in the ward, ICU, OR, and ER.

Early warning for capillary leakage - A MIS apparatus measuring (a rise in) tissue water would provide an early warning for increased capillary leakage, which potentially indicates sepsis, one of the largest causes of mortality in the ICU setting. Tissue water content could therefore be part of an 'early warning score' on the condition of the patient.

Provide guidance for fluid therapy in hospitalized patients - As stated above, hypovolemia is a common problem in many hospitalised patients and the first treatment of choice is fluid therapy. However, since fluids sometimes leak out of the vasculature, large volumes are required, potentially leading to a fluid overload. Fluid overload is common in the ward, ICU, and OR. Specific patient populations particularly at risk of fluid overload are septic patients due to leaky vasculature, patients with renal dysfunction, and patients undergoing heart surgery requiring a heart-lung machine (e.g., coronary artery bypass graft (CABG) surgery). A MIS apparatus measuring tissue water content could provide an early warning for fluid overload in patients receiving intravenous fluids since the first sign of fluid overload is tissue edema formation.

Provide guidance for haemodialysis - Patients with end stage kidney disease receive haemodialysis multiple times per week. However, when to stop a haemodialysis session is very ill-defined. When haemodialysis goes on too long, the patient will become dehydrated and hypovolemic which may remain unnoticed until the patient tries to stand up and (nearly) collapses. This risk is even higher in patients receiving haemodialysis at home. A MIS apparatus measuring tissue water content would provide guidance for haemodialysis for patients with chronic kidney disease.

Early warning for dehydration - Dehydration is a big issue amongst many home care patient populations, including babies, children in developed and developing countries, elderly, pregnant women, diabetics, and athletes, mountaineers, and soldiers. Especially in the neonatal and paediatric intensive care units, dehydration is a major problem which develops very rapidly. A MIS apparatus measuring tissue water content would provide an early warning for dehydration in these people/patients.

Edema formation at home - Several home care patient populations are at risk of developing peripheral edema, including patients with heart failure, nephrotic syndrome, liver cirrhosis, diabetes, hypertension and patients who had lymph surgery (e.g. as part of breast cancer surgery). Furthermore, pregnancies are often complicated by hypertension which also results in peripheral edema. A MIS apparatus measuring tissue water content would provide an early warning for edema formation in these patient populations. Especially for pregnant women, the early detection of leg edema could warn for pregnancy-induced hypertension, which besides peripheral edema can result in (pre-) eclampsia and premature birth.

There is therefore provided apparatus and methods for using MIS to monitor the fluid content of tissue in a human or animal body that are robust against movement of the apparatus and/or tissue, and/or robust against the low signal levels obtained from the tissue.

While the invention has been illustrated and described in detail in the drawings and foregoing description, such illustration and description are to be considered illustrative or exemplary and not restrictive; the invention is defined by the appended claims and is not limited to the disclosed embodiments.

Variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. Any reference signs in the claims should not be construed as limiting the scope.

## Claims

1. An apparatus (42) for using magnetic induction spectroscopy, MIS, to determine a measure of the fluid content of a tissue sample of a subject, the apparatus comprising:

   - a first excitation coil (46) that is to be placed near to the tissue sample for inducing a current in the tissue sample;
   - a receiver coil (48) that is to be placed near to the tissue sample so that the first excitation coil and the current in the tissue sample induce a current in the receiver coil, wherein the first excitation coil and the receiver coil are arranged in the same orientation with respect to each other;
   - a reference coil (58);
   - a second excitation coil (56) that is arranged close to the reference coil and that is for inducing a current in the reference coil, wherein the reference coil and the second excitation coil are arranged in the same orientation with respect to each other and are arranged such that the coupling between the second excitation coil and the reference coil matches the coupling between the first excitation coil and the receiver coil;
   wherein the receiver coil (48) is connected to the reference coil (58) such that the current induced in the receiver coil by the first excitation coil (46) and the current in the tissue sample flow in the opposite direction to the current induced in the reference coil by the second excitation coil (56); and
   - a control unit (50) that is configured to:

     - apply an alternating current to the first excitation coil and the second excitation coil;
     - obtain a measure of the current induced in the tissue sample using the output of the interconnected receiver coil and reference coil and a first signal, the first signal representing the alternating current applied to the first excitation coil and the second excitation coil; and
     - determine a measure of the fluid content of the tissue sample from the measure of the current induced in the tissue sample.

2. An apparatus (42) as claimed in claim 1, wherein the control unit (50) is configured to apply the same alternating current to each of the first excitation coil (46) and the second excitation coil (56).

3. An apparatus (42) as claimed in any of claims 1 or 2, wherein the first excitation coil (46) and the receiver coil (48) are configured to be placed around the tissue sample to be measured.

4. An apparatus (42) as claimed in any preceding claim, wherein the control unit (50) is configured to apply an alternating current having a first magnitude to the first excitation coil (46) and the second excitation coil (56) in order to measure the fluid content of the tissue sample at a first depth in the tissue sample, and wherein the control unit is further configured to:

   - apply a further alternating current having a second magnitude to the first excitation coil and the second excitation coil in order to measure the fluid content of the tissue sample at a second depth in the tissue sample;
   - obtain a measure of the current induced in the tissue sample at the second depth; and
   - determine a measure of the fluid content of the tissue sample at the second depth from the potential measured in the tissue sample at the second depth.

5. An apparatus (42) as claimed in any preceding claim, wherein the control unit (50) is further configured to:

   - control the apparatus to determine a plurality of measures of the fluid content of the tissue sample over time; and

- analyse the plurality of measures of the fluid content of the tissue sample over time to determine a measure of the heart rate, breathing rate and/or pulse pressure variations of the subject.

6. An apparatus (42) as claimed in any preceding claim, the apparatus further comprising a motion sensor that is for attachment to the subject and that is for measuring the movement of the subject; wherein the control unit (50) is further configured to:

- analyse the measurements of the movement of the subject from the motion sensor to determine if the amount of movement exceeds a threshold value; and
- discard the measure of the fluid content if the amount of movement exceeds the threshold value.

7. An apparatus (42) as claimed in any of claims 1-5, the apparatus further comprising a motion sensor that is for attachment to the subject and that is for measuring the movement of the subject; wherein the control unit (50) is further configured to:

- analyse the measurements of the movement of the subject from the motion sensor to determine if the amount of movement exceeds a threshold value; and
- only apply the alternating current to the first excitation coil (46) and the second excitation coil (56) if the amount of movement is below the threshold value.

8. A method of using magnetic induction spectroscopy, MIS, to determine a measure of the fluid content of a tissue sample of a subject, the method comprising:

- applying (203) an alternating current to a first excitation coil that is placed near to the tissue sample to induce a current in the tissue sample and a receiver coil that is placed near to the tissue sample and to a second excitation coil that is arranged close to a reference coil to induce a current in the reference coil, wherein the first excitation coil and the reciever coil are arranged in the same orientation with respect to each other, wherein the reference coil and the second excitation coil are arranged in the same orientation with respect to each other and are arranged such that the coupling between the second excitation coil and the reference coil matches the coupling between the first excitation coil and the receiver coil, and wherein the receiver coil is connected to the reference coil such that the current induced in the receiver coil by the first excitation coil and the current in the tissue sample flows in the opposite direction to the current induced in the reference coil by the second excitation coil;
- obtaining (205) a measure of the current induced in the tissue sample using the output of the interconnected receiver coil and reference coil and a first signal, the first signal representing the alternating current applied to the first excitation coil and the second excitation coil; and
- determining (207) a measure of the fluid content of the tissue sample from the measure of the current induced in the tissue sample.

9. A method as claimed in claim 8, wherein the step of applying (203) an alternating current comprises applying the same alternating current to each of the first excitation coil and the second excitation coil.

10. A method as claimed in claim 8 or 9, the method further comprising the step of placing (201) the first excitation coil and the receiver coil around the tissue sample to be measured.

11. A method as claimed in any of claims 8-10, wherein the step of applying (203) an alternating current to the first excitation coil and the second excitation coil comprises applying an alternating current having a first magnitude in order to measure the fluid content of the tissue sample at a first depth in the tissue sample; the method further comprising the steps of:

- applying a further alternating current having a second magnitude to the first excitation coil and the second excitation coil in order to measure the fluid content of the tissue sample at a second depth in the tissue sample; and
- obtaining a measure of the current induced in the tissue sample at the second depth; and determining a measure of the fluid content of the tissue sample at the second depth from the measure of the current induced in the tissue sample at the second depth.

12. A method as claimed in any of claims 8-11, the method further comprising the steps of:

- determining a plurality of measures of the fluid content of the tissue sample over time; and
- analysing the plurality of measures of the fluid content of the tissue sample over time to determine a measure of the heart rate, breathing rate and/or pulse pressure variations of the subject.

13. A method as claimed in any of claims 8-11, the method further comprising the steps of:

- measuring the movement of the subject;
- analysing the measurements of the movement of the subject to determine if the amount of movement exceeds a threshold value; and
one of the steps of:
discarding the measure of the fluid content if the amount of movement exceeds the threshold value.
- applying the alternating current to the first excitation coil and the second excitation coil only if the amount of movement is below the threshold value.

**Patentansprüche**

1. Gerät (42) für das Verwenden von Spektroskopie mit magnetischer Induktion (Magnetic Induction Spectroscopy - MIS), um ein Maß des Fluidgehalts einer Gewebeprobe eines Subjekts zu bestimmen, wobei das Gerät Folgendes umfasst:

- eine erste Erregerspule (46), die nahe der Gewebeprobe zum Induzieren eines Stroms in der Gewebeprobe zu platzieren ist;
- eine Empfängerspule (48), die nahe der Gewebeprobe zu platzieren ist, so dass die erste Erregerspule und der Strom in der Gewebeprobe einen Strom in der Empfängerspule induzieren, wobei die erste Erregerspule und die Empfängerspule in dieselbe Richtung in Bezug aufeinander eingerichtet sind;
- eine Referenzspule (58);
- eine zweite Erregerspule (56), die nahe der Referenzspule eingerichtet ist und zum Induzieren eines Stroms in der Referenzspule bestimmt ist, wobei die Referenzspule und die zweite Erregerspule in dieselbe Richtung in Bezug aufeinander eingerichtet sind, und derart eingerichtet sind, dass das Koppeln zwischen der zweiten Erregerspule und der Referenzspule mit dem Koppeln zwischen der ersten Erregerspule und der Empfängerspule übereinstimmt;
wobei die Empfängerspule (48) mit der Referenzspule (58) derart verbunden ist, dass der Strom, der in der Empfängerspule durch die erste Erregerspule (46) induziert wird, und der Strom in der Gewebeprobe in entgegengesetzte Richtung zu dem Strom fließen, der in der Referenzspule durch die zweite Erregerspule (56) induziert wird; und
- eine Steuereinheit (50) die konfiguriert ist, um:
- einen Wechselstrom an die erste Erregerspule und die zweite Erregerspule anzulegen;
- ein Maß des Stroms, der in der Gewebeprobe induziert wird, indem der Ausgang der verschalteten Empfängerspule und Referenzspule verwendet wird, und ein erstes Signal zu erhalten, wobei das erste Signal den Wechselstrom, der an die erste Erregerspule und die zweite Erregerspule angelegt wird, darstellt; und
- ein Maß des Fluidgehalts der Gewebeprobe aus dem Maß des Stroms, der in der Gewebeprobe induziert wird, zu bestimmen.

2. Gerät (42) nach Anspruch 1, wobei die Steuereinheit (50) konfiguriert ist, um den gleichen Wechselstrom an jede der ersten Erregerspule (46) und der zweiten Erregerspule (56) anzulegen.

3. Gerät (42) nach einem der Ansprüche 1 oder 2, wobei die erste Erregerspule (46) und die Empfängerspule (48) konfiguriert sind, um um die Gewebeprobe, die zu messen ist, platziert zu sein.

4. Gerät (42) nach einem der vorstehenden Ansprüche, wobei die Steuereinheit (50) konfiguriert ist, um einen Wechselstrom, der eine erste Größe aufweist, an die erste Erregerspule (46) und die zweite Erregerspule (56) anzulegen, um den Fluidgehalt der Gewebeprobe in einer ersten Tiefe in der Gewebeprobe zu messen, und wobei die Steuereinheit weiter konfiguriert ist, um:

- einen weiteren Wechselstrom, der eine zweite Größe aufweist, an die erste Erregerspule und die zweite Erregerspule anzulegen, um den Fluidgehalt der Gewebeprobe an einer zweiten Tiefe in der Gewebeprobe zu messen;

- ein Maß des Stroms, der in der Gewebeprobe in der zweiten Tiefe induziert wird, zu erhalten; und
- ein Maß des Fluidgehalts der Gewebeprobe in der zweiten Tiefe aus dem Potenzial, das in der Gewebeprobe in der zweiten Tiefe gemessen wird, zu bestimmen.

5. Gerät (42) nach einem der vorstehenden Ansprüche, wobei die Steuereinheit (50) weiter konfiguriert ist, um: das Gerät zu steuern, um eine Vielzahl von Messungen des Fluidgehalts der Gewebeprobe über Zeit zu bestimmen; und die Vielzahl von Maßen des Fluidgehalts der Gewebeprobe über Zeit zu analysieren, um ein Maß der Herzfrequenz, Atemfrequenz und/oder von Pulsdruckvariationen des Subjekts zu bestimmen.

6. Gerät (42) nach einem der vorstehenden Ansprüche, wobei das Gerät weiter einen Bewegungssensor umfasst, der zum Anbringen an dem Subjekt bestimmt ist, und der zum Messen der Bewegung des Subjekts bestimmt ist; wobei die Steuereinheit (50) weiter konfiguriert ist, um:

- die Messungen der Bewegung des Subjekts aus dem Bewegungssensor zu analysieren, um zu bestimmen, ob die Bewegungsmenge einen Schwellenwert überschreitet; und
- das Maß des Fluidgehalts zu verwerfen, falls die Bewegungsmenge den Schwellenwert überschreitet.

7. Gerät (42) nach einem der Ansprüche 1 bis 5, wobei das Gerät weiter einen Bewegungssensor umfasst, der zum Anbringen an dem Subjekt bestimmt ist, und der zum Messen der Bewegung des Subjekts bestimmt ist; wobei die Steuereinheit (50) weiter konfiguriert ist, um:

- die Messungen der Bewegung des Subjekts aus dem Bewegungssensor zu analysieren, um zu bestimmen, ob die Bewegungsmenge einen Schwellenwert überschreitet; und
- den Wechselstrom an die erste Erregerspule (46) und die zweite Erregerspule (56) lediglich anzulegen, falls die Bewegungsmenge unter dem Schwellenwert liegt.

8. Verfahren für das Verwenden von Spektroskopie mit magnetischer Induktion, um ein Maß des Fluidgehalts einer Gewebeprobe eines Subjekts zu bestimmen, wobei das Verfahren Folgendes umfasst:

- Anlegen (203) eines Wechselstroms an eine erste Erregerspule, die nahe der Gewebeprobe platziert ist, um einen Strom in der Gewebeprobe zu induzieren, und eine Empfängerspule, die nahe der Gewebeprobe platziert ist, und eine zweite Erregerspule, die nahe einer Referenzspule eingerichtet ist, um einen Strom in der Referenzspule zu erzeugen, wobei die erste Erregerspule und die Empfängerspule in dieselbe Richtung in Bezug aufeinander eingerichtet sind, wobei die Referenzspule und die zweite Erregerspule in dieselbe Richtung in Bezug aufeinander eingerichtet sind, und derart eingerichtet sind, dass die Kopplung zwischen der zweiten Erregerspule und der Referenzspule mit der Kopplung zwischen der ersten Erregerspule und der Empfängerspule übereinstimmt; und wobei die Empfängerspule mit der Referenzspule derart verbunden ist, dass der Strom, der in der Empfängerspule durch die erste Erregerspule induziert wird, und der Strom in der Gewebeprobe in entgegengesetzte Richtung zu dem Strom fließen, der in der Referenzspule durch die zweite Erregerspule induziert wird; und
- Erhalten (205) eines Maßes des Stroms, der in der Gewebeprobe induziert wird, indem der Ausgang der verschalteten Empfängerspule und Referenzspule verwendet wird, und eines ersten Signals, wobei das erste Signal den Wechselstrom, der an die erste Erregerspule und die zweite Erregerspule angelegt wird, darstellt; und
- Bestimmen (207) eines Maßes des Fluidgehalts der Gewebeprobe aus dem Maß des Stroms, der in der Gewebeprobe induziert wird.

9. Verfahren nach Anspruch 8, wobei der Schritt des Anlegens (203) eines Wechselstroms das Anlegen des gleichen Wechselstroms an jede der ersten Erregerspule und der zweiten Erregerspule umfasst.

10. Verfahren nach Anspruch 8 oder 9, wobei das Verfahren weiter den Schritt des Platzierens (201) der ersten Erregerspule und der Empfängerspule um die Gewebeprobe, die zu messen ist, umfasst.

11. Verfahren nach einem der Ansprüche 8 bis 10, wobei der Schritt des Anlegens (203) eines Wechselstroms an die erste Erregerspule und die zweite Erregerspule das Anlegen eines Wechselstroms umfasst, der eine erste Größe aufweist, um den Fluidgehalt der Gewebeprobe in einer ersten Tiefe in der Gewebeprobe zu messen; wobei das Verfahren weiter folgende Schritte umfasst:

- Anlegen eines weiteren Wechselstroms der eine zweite Größe aufweist, an die erste Erregerspule und die zweite Erregerspule, um den Fluidgehalt der Gewebeprobe an einer zweiten Tiefe in der Gewebeprobe zu messen; und

- Erhalten eines Maßes des Stroms, der in der Gewebeprobe in der zweiten Tiefe induziert wird; und Bestimmen eines Maßes des Fluidgehalts der Gewebeprobe in der zweiten Tiefe aus dem Maß des Stroms, der in der Gewebeprobe in der zweiten Tiefe induziert wird.

**12.** Verfahren nach einem der Ansprüche 8 bis 11, wobei das Verfahren weiter die folgenden Schritte umfasst:

- Bestimmen einer Vielzahl von Maßen des Fluidgehalts der Gewebeprobe über Zeit; und
- Analysieren der Vielzahl von Maßen des Fluidgehalts der Gewebeprobe über Zeit, um ein Maß der Herzfrequenz, Atemfrequenz und/oder von Pulsdruckvariationen des Subjekts zu bestimmen.

**13.** Verfahren nach einem der Ansprüche 8 bis 11, wobei das Verfahren weiter die folgenden Schritte umfasst:

- Messen der Bewegung des Subjekts;
- Analysieren der Messungen der Bewegung des Subjekts, um zu bestimmen, ob die Bewegungsmenge einen Schwellenwert überschreitet; und

einen der folgenden Schritte:

- Verwerfen des Maßes des Fluidgehalts, falls die Bewegungsmenge den Schwellenwert überschreitet.
- Anlegen des Wechselstroms an die erste Erregerspule und die zweite Erregerspule lediglich, falls die Bewegungsmenge unter dem Schwellenwert liegt.

**Revendications**

**1.** Appareil (42) destiné à l'utilisation de la spectroscopie à induction magnétique, MIS, pour déterminer une mesure de la teneur en fluide d'un échantillon de tissu d'un sujet, l'appareil comprenant :

- une première bobine d'excitation (46) qui doit être placée près de l'échantillon de tissu pour induire un courant dans l'échantillon de tissu ;
- une bobine réceptrice (48) qui doit être placée près d'un échantillon de tissu de sorte que la première bobine d'excitation et le courant dans l'échantillon de tissu induisent un courant dans la bobine réceptrice, dans lequel la bobine d'excitation et la bobine réceptrice sont agencées selon la même orientation l'une par rapport à l'autre ;
- une bobine de référence (58) ;
- une seconde bobine d'excitation (56) qui est agencée près de la bobine de référence et qui est destinée à l'induction d'un courant dans la bobine de référence, dans lequel la bobine de référence et la seconde bobine d'excitation sont agencées selon la même orientation l'une par rapport à l'autre et sont agencées de sorte que le couplage entre la seconde bobine d'excitation et la bobine de référence correspond au couplage entre la première bobine d'excitation et la bobine réceptrice ;
dans lequel la bobine réceptrice (48) est reliée à la bobine de référence (58) de sorte que le courant induit dans la bobine réceptrice par la première bobine d'excitation (46) et le courant dans l'échantillon de tissu circulent dans la direction opposée au courant induit dans la bobine de référence par la seconde bobine d'excitation (56) ; et
- une unité de commande (50) qui est configurée pour :
- appliquer un courant alternatif à la première bobine d'excitation et à la seconde bobine d'excitation ;
- obtenir une mesure du courant induit dans l'échantillon de tissu en utilisant le résultat de la bobine réceptrice et de la bobine de référence interconnectées et un premier signal, le premier signal représentant le courant alternatif appliqué sur la première bobine d'excitation et sur la seconde bobine d'excitation ; et
- déterminer une mesure de la teneur en fluide de l'échantillon de tissu à partir de la mesure du courant induit dans l'échantillon de tissu.

**2.** Appareil (42) selon la revendication 1, dans lequel l'unit de commande (50) est configurée pour appliquer le même courant alternatif à chacune de la première bobine d'excitation (46) et de la seconde bobine d'excitation (56).

**3.** Appareil (42) selon l'une quelconque des revendications 1 ou 2, dans lequel la première bobine d'excitation (46) et la bobine réceptrice (48) sont configurées pour être placées autour de l'échantillon de tissu à mesurer.

**4.** Appareil (42) selon l'une quelconque des revendications précédentes, dans lequel l'unité de commande (50) est configurée pour appliquer un courant alternatif présentant une première magnitude à la première bobine d'excitation (46) et à la seconde bobine d'excitation (56) de façon à mesurer la teneur en fluide de l'échantillon de tissu au niveau d'une première profondeur de l'échantillon de tissu, et dans lequel l'unité de commande est en outre configurée pour :

- appliquer un courant alternatif supplémentaire présentant une seconde magnitude à la première bobine d'excitation et à la seconde bobine d'excitation de façon à mesurer la teneur en fluide de l'échantillon de tissu au niveau d'une seconde profondeur de l'échantillon de tissu ;
- obtenir une mesure du courant induit dans l'échantillon de tissu au niveau d'une seconde profondeur ; et
- déterminer une mesure de la teneur en fluide de l'échantillon de tissu au niveau d'une seconde profondeur à partir du potentiel mesuré dans l'échantillon de tissu au niveau de la seconde profondeur.

**5.** Appareil (42) selon l'une quelconque des revendications précédentes, dans lequel l'unité de commande (50) est en outre configurée pour :

- commander un appareil pour déterminer une pluralité de mesures de la teneur en fluide de l'échantillon de tissu au fil du temps ; et
- analyser la pluralité de mesures de la teneur en fluide de l'échantillon de tissu au fil du temps pour déterminer une mesure de la fréquence cardiaque, de la fréquence respiratoire et/ou des variations de pression artérielle du sujet.

**6.** Appareil (42) selon l'une quelconque des revendications précédentes, l'appareil comprenant en outre un capteur de mouvement qui est destiné à être attaché au sujet et qui est destiné à la mesure du mouvement du sujet ; dans lequel l'unité de commande (50) est en outre configurée pour :

- analyser les mesures du mouvement du sujet à partir du capteur de mouvement pour déterminer si la quantité de mouvement excède une valeur seuil ; et
- écarter la mesure de la teneur en fluide si la quantité de mouvement excède la valeur seuil.

**7.** Appareil (42) selon l'une quelconque des revendications 1 à 5, l'appareil comprenant en outre un capteur de mouvement qui est destiné à être attaché au sujet et qui est destiné à la mesure du mouvement du sujet ; dans lequel l'unité de commande (50) est en outre configurée pour :

- analyser les mesures du mouvement du sujet à partir du capteur de mouvement pour déterminer si la quantité de mouvement excède une valeur seuil ; et
- appliquer le courant alternatif à la première bobine d'excitation (46) et à la seconde bobine d'excitation (56) seulement si la quantité de mouvement est en dessous de la valeur seuil.

**8.** Procédé d'utilisation d'un spectroscope à induction magnétique, MIS, pour déterminer une mesure d'une teneur en fluide d'un échantillon de tissu d'un sujet, le procédé comprenant :

- l'application (203) d'un courant alternatif à une première bobine d'excitation qui est placée près de l'échantillon de tissu pour induire un courant dans l'échantillon de tissu et une bobine réceptrice qui est placée près de l'échantillon de tissu et de la seconde bobine d'excitation qui est agencée près d'une bobine de référence pour induire un courant dans la bobine de référence, dans lequel la première bobine d'excitation et la bobine réceptrice sont agencées selon la même orientation l'une par rapport à l'autre, dans lequel la bobine de référence et la seconde bobine d'excitation sont agencées selon la même orientation l'une par rapport à l'autre et sont agencée de sorte que le couplage entre la seconde bobine d'excitation et la bobine de référence correspond au couplage entre la première bobine d'excitation et la bobine réceptrice, et dans lequel la bobine réceptrice est reliée à la bobine de référence de sorte que le courant induit dans la bobine réceptrice par la bobine de référence et par le courant dans l'échantillon de tissu circule dans la direction opposée au courant induit dans la bobine de référence par la seconde bobine d'excitation ;
- l'obtention (205) d'une mesure du courant induit dans l'échantillon de tissu en utilisant le résultat de la bobine réceptrice et de la bobine de référence interconnectée et un premier signal, le premier signal représentant le courant alternatif appliqué à la première bobine d'excitation et à la seconde bobine d'excitation ; et
- la détermination (207) d'une mesure de la teneur en fluide de l'échantillon de tissu à partir de la mesure du courant induit dans l'échantillon de tissu.

9. Procédé selon la revendication 8, dans lequel l'étape d'application (203) d'un courant alternatif comprend l'application du même courant alternatif à chacune de la première bobine d'excitation et de la seconde bobine d'excitation.

10. Procédé selon la revendication 8 ou 9, le procédé comprenant en outre l'étape de placement (201) de la première bobine d'excitation et de la bobine réceptrice autour de l'échantillon de tissu à mesurer.

11. Procédé selon l'une quelconque des revendications 8 à 10, dans lequel l'étape d'application (203) d'un courant alternatif à la première bobine d'excitation et à la seconde bobine d'excitation comprend l'application d'un courant alternatif présentant une première magnitude de façon à mesurer la teneur en fluide de l'échantillon de tissu au niveau d'une première profondeur dans l'échantillon de tissu ;
le procédé comprenant en outre les étapes consistant à :

   - appliquer un courant alternatif supplémentaire présentant une seconde magnitude à la première bobine d'excitation et à la seconde bobine d'excitation de façon à mesurer la teneur en fluide de l'échantillon de tissu au niveau d'une seconde profondeur dans l'échantillon de tissu ; et
   - obtenir d'une mesure du courant induit dans l'échantillon de tissu au niveau de la seconde profondeur ; et déterminer une mesure de la teneur en fluide de l'échantillon de tissu au niveau de la seconde profondeur à partir de la mesure du courant induit dans l'échantillon de tissu au niveau de la seconde profondeur.

12. Procédé selon l'une quelconque des revendications 8 à 11, le procédé comprenant en outre les étapes consistant à :

   - déterminer une pluralité de mesures de la teneur en fluide de l'échantillon de tissu au fil du temps ; et
   - analyser la pluralité de mesures de la teneur en fluide de l'échantillon de tissu au fil du temps pour déterminer une mesure de la fréquence cardiaque, de la fréquence respiratoire et/ou des variations de pression artérielle du sujet.

13. Procédé selon l'une quelconque des revendications 8 à 11, le procédé comprenant en outre les étapes consistant à :

   - mesurer le mouvement du sujet ;
   - analyser les mesures du mouvement du sujet pour déterminer si la quantité de mouvement excède une valeur seuil ; et
   une des étapes consistant à :
   écarter la mesure de la teneur en fluide si la quantité de mouvement excède la valeur seuil.
   - appliquer le courant alternatif à la première bobine d'excitation et à la seconde bobine d'excitation seulement si la quantité de mouvement est en dessous de la valeur seuil.

Figure 1 (Prior art)

(a)

26

32
Excitation signal (I, f)

Reference signal

34
Processing Unit

σ

24

22

30

(b)

28

26

32
Excitation signal (I, f)

Reference signal

34
Processing Unit

σ

24

22

30

Figure 2 (Prior art)

(c)

Figure 2 (Prior art)

Figure 3

Figure 4

201 — Place excitation coil and receiver coil adjacent to a tissue sample to be measured

203 — Apply an alternating current of magnitude I and frequency f to the excitation coil and a second excitation coil

205 — Obtain a signal from the interconnected receiver coil and reference coil

207 — Determine a measure of the fluid content using the measured signal

Figure 5

Figure 6

Figure 7

Figure 8

62

66

72

74

64

Excitation
signal (I, f)

Reference
signal

Processing
Unit

σ

70

Figure 9

301 — Place excitation coil around the tissue sample to be measured

303 — Apply an alternating current of magnitude I and frequency f to the excitation coil

305 — Measure the change in impedance of the excitation coil over time

307 — Determine a measure of the fluid content using the measured change in impedance

Figure 10

62

68

66

64

72

Excitation
signal (I, f)

74

Processing
Unit

→ σ

Reference
signal

70

Figure 11

62

68

76    66

64

72

Excitation
signal (I, f)

74

Processing
Unit

→ σ

70

Figure 12

Figure 13

401 — Place excitation coil adjacent to a tissue sample to be measured and place electrodes at the measurement site

403 — Apply an alternating current of magnitude I and frequency f to the excitation coil

405 — Measure the potential generated in the tissue sample using the electrodes

407 — Determine a measure of the fluid content of the tissue sample using the measured potential

Figure 14

Figure 15

Figure 16

Figure 17

501 — Obtain a received signal using receiver components

503 — Obtain a reference signal

505 — Apply a phase adjustment to one of the signals to account for differences in form of the receiver and reference components

507 — Determine the phase difference between the signals

509 — Determine a measure of the fluid content/tissue conductivity from the determined phase difference

Figure 18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20100127705 A

**Non-patent literature cited in the description**

- **ZULKARNAY ZAKARIA et al.** Advancements in Transmitters and Sensors for Biological Tissue Imaging in Magnetic Induction Tomography. *Sensors,* 29 May 2012, vol. 12 (12), 7126-7156
- **ZHENG XU et al.** A multi-channel magnetic induction tomography measurement system for human brain model imaging. *PHYSIOLOGICAL MEASUREMENT,* 02 June 2009, vol. 30 (6), S175-S186
- **GONZALEZ et al.** Over-Hydration Detection in Brain by Magnetic Induction Spectroscopy. *International Conference on Electrical Bioimpedance, Journal of Physics: Conference Series,* 2010, 224